**Europäisches Patentamt**

**European Patent Office**

**Office européen des brevets**

(19)

(11) Veröffentlichungsnummer: **0 307 566 B1**

(12) **EUROPÄISCHE PATENTSCHRIFT**

(45) Veröffentlichungstag der Patentschrift: **20.01.93**

(51) Int. Cl.⁵: **G01N 33/487**

(21) Anmeldenummer: **88111013.4**

(22) Anmeldetag: **10.07.88**

(54) **Verfahren zur kristallmorphologischen Blut- und Harnanalyse, zur Frühdiagnose und zur Herstellung von Arzneimitteln.**

(30) Priorität: **28.07.87 DE 3724988**

(43) Veröffentlichungstag der Anmeldung:
**22.03.89 Patentblatt 89/12**

(45) Bekanntmachung des Hinweises auf die
Patenterteilung:
**20.01.93 Patentblatt 93/03**

(84) Benannte Vertragsstaaten:
**AT BE CH DE ES FR GB GR IT LI LU NL SE**

(56) Entgegenhaltungen:
**WO-A-86/04141**
**DE-A- 825 906**
**FR-A- 2 057 343**
**US-A- 4 183 729**
**US-A- 4 711 697**

(73) Patentinhaber: **HEINZ SPAGYRIK INSTITUT AG**

**CH-Corticiasca (TI)(CH)**

(72) Erfinder: **Ullrich, Jürgen Heinz, Prof. Dr. Dr.**
**Verdistrasse 11**
**I-39012 Meran/Bz.(IT)**

(74) Vertreter: **Jahn-Held, Wilhelm W. Dr.Dr.-Ing.**
**Dipl.-Chem.**
**Schöne Aussicht 8**
**W-3513 Staufenberg-Landwehrhagen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur kristallmorphologischen Blut- und Harnanalyse zur Frühdiagnose und zur Herstellung von Arzneimitteln.

R. Wernik, "Das Beste aus Readers Digest", September 1984, Seite 27-31, trifft in dieser Veröffentlichung über:" Das Blut, Spiegel unserer Geschichte", folgende Aufgabenstellung: " Vielleicht wird es den Ärzten der Zukunft dank den in einem einzigen Blutstropfen enthaltenen Informationen einmal gelingen, jedem Kranken eine massgeschneiderte Therapie zu verordnen".

Diese Aufgabe zu lösen, ist eine Aufgabe des Verfahrens der Erfindung.

Die Wo- A- 8604142 beschreibt ein Verfahren zur Herstellung von Kristallbildern zur Erkennung von Krankheiten durch optischen Analogievergleich zu in gleicher Weise erzeugten, charakteristischen Typen solcher Kristallbilder, wobei aus der wässrigen Phase von Vollblut oder von Sekret ein Destillat und aus dem getrockneten Rückstand ein Kalzinat bei gesteigerter Temperatur bis 750°C und danach bis zur Sinterung bei 1150°C erzeugt wird. Es wird danach das Kalzinat mit dem Destillat vermischt und von diesem Gemisch werden einige Tropfen auf einen Objektträger in 15 - 50 min bei 17- 28°C und bei einer relativen Feuchtigkeit von 40-80% zur Bildung eines Kristallisates gebracht. Dieser Stand der Technik führt nur zur Erzeugung von charakteristischen Typen von Kristallbildern und nicht zu Mutterformen und davon abgeleiteten Tochterformen von Kristalltexturen und Kristallformen mit erheblicher Breite. und Genauigkeit der Darstellung.

Es ist weiter die Aufgabe der Erfindung, hochsensible und hochspezifische Diagnoseformen zur ganzheitlichen Darstellung der pathologischen und physiologischen Vorgänge im menschlichen und tierischen Körper als Frühdiagnose der kleinsten Veränderungen zu entwickeln, die in diesem Stadium mit röntgenologischen, serologischen, sonographischen, enzymatischen Methoden noch nicht festzustellen sind.

Die Lösungen dieser Aufgaben der Erfindung sind im kennzeichnenden Teil des Patentanspruches 1 definiert.

Diese Lösung ist durch die folgenden Verfahrensstufen definiert:

1) Vermischen des menschlichen Blutes oder Harn oder des tierischen Blutes oder Harn von Warmblütern mit mindestens 3-fach destilliertem Wasser im Volumenverhältnis von 1: 2,5 bis 3,5, vorzugsweise von 1: 2,7 bis 3,2, und drucklose Destillation bei Siedetemperatur bis zur Bildung eines Destillates und eines praktisch trockenen Blutkuchens oder Harnsedimentes,

2) Kalzination des trockenen Blutkuchens oder Harnsedimentes linear ansteigend bis auf 600°C bei bis maximal 1 % Streuung in einer Aufheizzeit von 60 bis 70 min, vorzugsweise von 65 min, bei konstanter Heizleistung, langsame Abkühlung des Kalzinates bis auf etwa 150°C in etwa 2 bis 4 h, Pulverisierung des abgekühlten Kalzinates,

3) Vermischen des Destillates aus Stufe 1 und des Kalzinates aus Stufe 2, Einschalten einer Ruhezeit von etwa 8 bis 12 h zur Bildung einer homogenen Phase, Filtration der flüssigen Mischung,

4) Aufbringen der gefilterten Lösung mit 19,9 bis 20,1 °C, vorzugsweise mit 20,0° C, bei einer relativen Luftfeuchtigkeit von etwa 40 bis 60 %, auf gläserne, mikroskopische Objektträger in Form von 2 Tropfen mit einem Durchmesser von 6 bis 10 mm, vorzugsweise von 8 mm, Verdunsten des Wassers bei 19,9 bis 20,1 °C unter Ausbildung von praktisch trockenen Kristalltexturen mit Kristallformen und deren Zuordnung zu definierten menschlichen oder tierischen Organen oder physiologischen Funktionskreisen oder Keimblattsektoren, derart, dass dem entodermalen Bereich (A) eine bevorzugte Teilfläche in radialer Richtung im äusseren, konzentrischen Ringraum, und dem mesodermalen Bereich (B) eine bevorzugte Teilfläche in radialer Richtung im mittleren, konzentrischen Ringraum und dem ektodermalen Bereich (C) der zentrale Ringraum zugeordnet ist,

5) Mikroskopische Ermittlung der Kristalltexturen und Kristallformen im Dunkelfeld mit

| Vergrösserungen: | zur Darstellung: |
|---|---|
| 10 bis 15- fach | der Sektoren im Gesamtbild, |
| 20 bis 50- fach | der einzelnen Sektoren (A), oder (B), oder (C) nach Stufe 4, |
| 60 bis 160- fach | der kompakten, flächenhaften und/oder räumlichen Dendriten als Texturen, |
| 170 bis 800-fach | der Dendritbilder mit morphologischen, geometrischen, räumlichen Grundformen zur Identifierung der kristallbildenden Grundformen, |

6) mikroskopischer und fotooptischer Analogievergleich der in Stufe 5 definierten Kristalltexturen und Kristallformen mit solchen eines gesicherten, pathologischen Zustandes aus Vergleichsserien als Standardtypen oder mit den Koordinaten von Mutter- und Tochterformen zur Diagnose des Gesundheits- oder des Krankheitszustandes von Mensch oder von Wirbel- und Säugetieren, Die alternative und bevorzugte Ausgestaltung des Verfahrens der Erfindung ist in den Unteransprüchen definiert.

Diese betreffen die Menge des in Stufe 1 einzusetzenden Blutes oder Harns, und bestimmte Massnahmen bei der Durchführung der Kalzination in der Stufe 2.

Diese Ausgestaltung des Verfahrens der Erfindung betrifft auch die geometrische und kristallmorphologische Ausbildung der Kristalltexturen und Kristallformen der Mutterformen und der Tochterformen in der Fixierung der biologischen Koordinaten in der beschreibenden Definition von mikroskopisch und fotooptisch erfasster Figuren.

Diese Ausgestaltung betrifft auch geometrische und kristallmorphologische Figuren als biologische Koordinaten als Kristalltexturen und Kristallformen, die aus mehreren Mutter- und/oder Tochterformen abgeleitet sind.

Diese alternative Ausgestaltung betrifft auch die Kristallisierung von Texturen und Formen in 3 definierten Bereichen aus konzentrischen Kreisen, denen in diesen Bereichen Organe oder Organzonen in bestimmter, definierter Reihenfolge von aussen nach innen zugeordnet sind.

Das Verfahren der Erfindung verwendet folgende Definitionen:

Unter Kristalltexturen werden die bei der Kristallisation in Stufe 4 erzeugten, flächenhaften Aggregate der Kristallpartikel bzw. - Nadeln verstanden.

Unter Kristallformen werden Grund-, Mutter-, Tochter- Formen verstanden, die eine eindeutige Identifizierung der phänomenalen Figuren gestatten.

Unter Grundformen werden einfache, geometrische, nicht mehr reduzierbare Figuren als Elemente verstanden. Unter Mutterformen werden Grundformen, oder deren Ableitungen, oder einheitlich abgeschlossene Figuren, oder einheitliche, abgeschlossene und identifizierbare Kristallformen verstanden.

Unter Tochterformen werden aus den Mutterformen abgeleitete Formen als Kristalltexturen verstanden. Bei der Kristallisation nach Stufe 4 können sich freie Kristallstrukturen bilden, die untereinander keine systematische Ordnung bilden, wie bei einer Gitterstruktur.Es kann die Lagerichtung einzelner Partikel zueinander unregelmässig sein. Solche Partikel können sich kreuzen oder nicht berühren.

Unter Figuren werden typische,reproduzierbare, definierte Kristalltexturen und Kristallfiguren verstanden, die mikroskopisch und fotooptisch sichtbar sind, und die mathematisch und beschreibend gekennzeichnet und dadurch definiert sind. Unter Reproduzierbarkeit der Kristalltexturen und Kristallformen wird die sichtbare, figürliche Formengleichheit oder Formenähnlichkeit bei mehrfacher, zeitlich unabhängiger Wiederholung der Erzeugung der Kristallisate aus der homogenen Mischung des Destillates und des löslichen Teiles des Kalzinates aus mehreren Tropfen aus der gleichen oder vergleichbaren Ausgangssubstanz verstanden.

Es ist dabei zu beachten, dass Blut mit den Organen und Organgeweben im ständigen Gleichgewicht steht bis zur Entnahme der für die Durchführung benötigten Menge.Dies gilt auch für Harn unter Berücksichtung der durch die Funktion der Nieren sich ergebenden Spezialisierung der Ausscheidung.

Die Vergleiche der Kristalltexturen aus Harn mit denen aus klinisch gesicherten Befunden ergeben übereinstimmende Ergebnisse in Form, Aufbau und Information.

In diesen zeitlichen Abständen der Entnahme finden im biologischen, dynamischen Prozess des lebenden Individuums laufend Veränderungen in den Gleichgewichten statt. Dies bedeutet, dass die sichtbar gemachten Kristalltexturen und Kristallformen im Gesamtbild Änderungen zeigen. Die Reproduzierbarkeit bleibt jedoch für die charakteristischen Mutter- und Tochterformen erhalten. Es ist dadurch die Regel zum vollständigen, technischen Handeln auch für diese Entnahme von zu analysirende Blut- oder Harnmengen gegeben. Unter Blut wird das Venenblut von Menschen oder von Warmblütern verstanden.

Das Verfahren der Erfindung wird durch das folgende Ausführungsbeispiel erläutert.

Es werden 10 ml Venenblut einer Frau mit 30 ml 3-fach destilliertem Wasser vermischt und bei 96°C mit Luftkühlung überdestilliert.

Die Destillation ist beendet, wenn der Blutrückstand im Destillationskolben praktisch trocken ist,also kein Wasser mehr abgibt unter den gleichen Arbeitsbedingungen.

In der folgenden Verfahrensstufe wird der Rückstand in einem Porzellantiegel auf 200°C erwärmt und dabei getrocknet. Danach wird der Rückstand auf 400°C erwärmt, wobei sich geruchsintensive Gase bilden. Danach wird der Rückstand auf 600°C erwärmt und damit verascht. Die Erwärmung und Veraschung als Kalzination erfolgt in einem geschlossenen, regelbaren Muffelofen ohne Zufuhr von Luft als Sauerstoff. Der kalzinierte Rückstand wird danach im Ofen auf 150°C abgekühlt, danach entnommen und im Mörser pulverisiert.

In der folgenden Verfahrensstufe wird das Destillat aus Stufe 1 mit dem kalzinierten Blutkuchen aus Stufe 2 vermischt. Das Gemisch beider Komponenten erhält bei 20°C Umgebungstemperatur eine Ruhezeit von 8 h zur Bildung einer homogenen Phase. Es wird danach das Gemisch durch mikrofeines Filterpapier filtriert.

In der folgenden Verfahrensstufe wird die gefilterte Lösung bei 20°C geregelter Temperatur auf einen gläsernen, mikroskopischen Objektträger in 2 Tropfen von je 0,08 ml aufgebracht, die einen Durchmesser von 8 mm bilden, und in etwa 20 min das Wasser verdunstet. Dabei erfolgt die Ausbildung von Kristalltexturen und Kristallformen.

In der folgenden Verfahrensstufe wird im mikroskopischen Dunkelfeld mit stufenweiser Vergrösserung auf das 12,5- , 25-, 160-, 400-fache die reproduzierbare und interpretierbare Analyse des Blutbildes durchgeführt. Diese mikroskopischen Bilder werden fotografiert zur Dokumentation.

In der folgenden Verfahrensstufe erfolgt der Analogievergleich der definierten Texturen und Formen des Blutbildes mit solchen eines gesicherten pathogenen Zustandes zur Ermittlung des Krankheitszustandes.

Dieser Analogievergleich kann beispielsweise auch mit Standardtypen aus Vergleichsserien erfolgen, um den physiologischen, metabolischen und pathologischen Zustand zu bestimmen.

In einer weiteren Verfahrensstufe wird aus 7 ml der filtrierten Lösung unter Zugabe von 23 ml isotonischer, physiologischer Lösung und von 20 ml 40%- igem Äthylalkohol das Autovaxin hergestellt. Dieses Heilmittel hat den Handelsnamen "Homodot".

Bei weniger oder mehr als 7 ml filtrierter Lösung beträgt das Mischungsverhältnis von filtrierter Lösung : isotonischer, physiologischer Lösung : Äthylalkohol ( 40- Vol.-%) = 1 : 5 : 4.

In einer anderen Variante dieser weiteren Verfahrensstufen wird dem Venenblut dieser Frau ein Tropfen entnommen, und dieser auf Blutagarboden geimpft und bei einer Brüttemperatur von 37°C ein Antivaxin von 2 ml nach 38 h abgezogen.

Diese Masse wird dann mit 20 ml 3-fach destilliertem Wasser vermischt und danach destilliert. Der Rückstand wird danach gemäss der Verfahrensstufe 2 bei Temperaturen bis 200°, 400°, 600°C kalziniert und danach pulverisiert und mit dem Destillat vermischt. Nach einer Ruhezeit von 2 h wird danach filtriert. Das Filtrat von 6 ml wird mit 14 ml physiologischer Lösung und mit 20 ml 40-%- igem Äthlylalkohol vermischt.

Diese mikrobiologisch gezüchtete Autovaxin stelt ein nicht toxisches, proteinfreies, nicht allergenes Heilmittel dar.

Dieses Heilmittel hat den Handelsnamen "Antihomodot". Dieses wird zur spezifischen Simulation chronischer, toxischer und parasitogener Herde im Körper dieser Frau verwendet.

Die Verdünnung des Blutes mit Wasser in Stufe 1 erfolgt, um genügend Dampfdruck für die Destillation zu erzeugen und um genügend Lösung zu haben, in welcher das Kalzinat aus Stufe 2 in Stufe 3 zu einer homogenen Phase gelöst wird.

Das Verhältnis von 1: 2,5 bis 3,5 stellt eine Auswahl dar. Wenn weniger als das 2,5- fache eingestellt wird, dann ensteht insbesondere bei hochviskosem Blut, zu wenig Dampfdruck und damit zu wenig Destillat.

Sofern über das 3,5- fache bei niedrigviskosem Blut eingesetzt wird, entsteht ein zu hoher Flüssigkeitsanteil im Kristallisat in Stufe 4 und damit eine ungenügende Ausbildung der Kristalltexturen.

Eine weitere Auswahl stellt in Stufe 2 die einzustellende Endtemperatur von 600°C dar.Die Einhaltung der Temperatursteigerung gemäss Stufe 2 ergibt eine optimale Ausprägung des Kristallisates in Stufe 4.Bei zu geringer Temperatur wird das Kristallisat grobflächig und bei zu hoher Temperatur zu feinkörnig und führt somit zu einer ungenügenden Ausbildung der Formen.

Die Analyse der Kristalltexturen erfolgt in Stufe 5 im mikroskopischen Dunkelfeld. Mit der 10 - 15- fachen Vergrösserung wird ein Gesamtbild zur Abgrenzung der Sektoren (A), (B), (C) erzeugt.

Mit der 20 - 50- fachen Vergrösserung wird an der linken Abszisse als Scheitelpunkt ein Bildausschnitt und ein Bildpunkt in Übereinstimmung gebracht und optisch als Sektor (A) erfasst zur analytischen Auswertung.

In der gleichen Weise werden die Sektoren (B), und (C) ermittelt und analytisch ausgewertet.

Mit der 60 - 160- fachen Vergrösserung werden innerhalb der Sektoren als Bereiche die kompakten, flächenhaften und/ oder räumlichen Dendriten als Kristalltexturen und Kristallformen analytisch ermittelt. Diese werden dann nach ihrer optischen Isolierung organbezogen diagnostisch ausgewertet.

Mit der 180 - 800-fachen Vergrösserung erfolgt die Darstellung und Identifizierung der morphologischen, geometrischen, räumlichen Grundformen.

Diese Grundformen sind als definierte Figuren auf biologisch relevante Kationen bezogen, insbesondere auf Na, K, Mg, Ca.Diese Grundformen zeigen dominante Stoffwechselprozesse als Ausdruck einer Übersättigung des Gewebes.

Es werden daraus pathogene Stoffwechselzustände diagnostiziert.

In Stufe 6 ergibt die mikroskopische und fotooptische Auswertung als Analogievergleich der in Stufe 5 definierten Kristalltexturen und Kristallformen eine gesicherte Diagnose des Gesundheits- oder Krankheitszustandes von Mensch oder Tier.Solche pathologischen Zustände sind beispielsweise chronische, rheumatische Leukämie.

Es wird nach dem Verfahren der Erfindung aus diesem gesicherten, pathologischen Zustand die figürliche Abbildung der Kristalltexturen und Kristallformen hergestellt. Durch die Übereinstimmung der Mutter-, Tochter-, Grund-Formen aus den Kristalltexturen und Formen der figürlichen Darstellung der erkrankten Person mit denen aus der figürlichen Darstellung aus dem gesicherten, pathologischen Zustand wird durch diese Zuordnung der pathologische Krankheitszustand der untersuchten Person zweifelsfrei diagnostiziert.

Es ergeben beispielsweise 10 verschiedene Kristallisate von Leukämie untersuchter Personen gleiche, gemeinsame, charakteristische Merkmale in den Mutter-, Tochter-Grund- Formen neben unterschiedlichen Figuren aus den unterschiedlichen Zuständen der Erkrankungen dieser Personen.

Die alternative Durchführung der Kalzination nach Anspruch 3 erfolgt stufenweise linear um durch diese Massnahme die Rauchentwicklung in den Stufen zu steuern.

Das Verfahren der Erfindung wird durch Figuren der Mutter- und Tochterformen der Kristalltexturen und Kristallformen in tabellarischer Aufstellung mit biologischen Koordinaten beschrieben.

Die Mutterformen sind auf der Ordinate bei der Abszisse " 0 " dargestellt.

Die Tochterformen sind auf den Abszissen 01- 27 zu den Ordinaten A 01 - Z 4 30 dargestellt.

Diese figürliche Darstellung entspricht der mikroskopischen und fotooptischen Auswertung der Kristalltexturen und Kristallformen von klinisch gesicherten Krankheitszuständen in mehrfacher Wiederholung.

| Figur | Darstellung : Mutter-und Tochterformen : | |
| --- | --- | --- |
| | Abszisse $y =$ | Ordinate $x =$ |
| 1 | A 01- E 05<br>N 14-P 16 | 0 – 07 |
| 2 | " | 08-15 |
| 3 | " | 16-23 |
| 4 | " | 24-27 |
| 5 | F 06- M 13 | 0-07 |
| 6 | " | 08-15 |
| 7 | " | 16-23 |
| 8 | " | 24-27 |
| 9 | Q 17- X 24 | 0-07 |
| 10 | " | 08-15 |
| 11 | " | 16-23 |
| 12 | " | 25-27 |
| | Mischformen: | |
| 13 | Y 25- Z4 30 | 0-07 |
| 14 | " | 08-15 |
| 15 | " | 16-23 |

Das Verfahren der Erfindung wird weiter beispielsweise erläutert.
Beispiel für Abbildungen mit den Vergrösserungen nach Stufe 5.

| Abbildung | Darstellung |
|---|---|
| 1 | Vergrösserung 12,5 fach: Gesamtbild der Kristall- texturen, |
| 2 | Vergrösserung 25- fach: Abbildung der Sektoren, |
| 3 | Vergrösserung 65- fach: Flächenhafte Abbildung der Dendriten- Form, |
| 4 | Vergrösserung 160-fach: Flächenhafte Abbildung der geometrischen Grundform. |

Beispiele für den Analogievergleich des Blutes eines Mannes nach Stufe 6 mit den Koordinaten eines gesicherten, pathologischen Zustandes zur Diagnose des Krankheitszustandes.

Durch optischen Vergleich der Kristallstrukturen und Kristallformen sind die folgenden Koordinaten für das Blut des Erkrankten ermittelt:

| Abbildung | Koordinaten |
|---|---|
| 5 | Bereich (A): Sektor: D 04- 0 ( C 03 - 13) + B 03- 0 + D 04- 014, Bereich (B): Sektor D 04- 04 + D 04- 11 + K 11 -12 Bereich (C): Sektor $Z_1$27 - 01 ( K 11-12 + K11-13 ( K 11- 12 + K 11- 13 + K 11 -09) |

| Abbildung | Koordinaten |
|---|---|
| 6 | Bereich (A): Sektor: |
| | E 05- 11+ E 05-22 + N 14-03, |
| | Bereich (B): Sektor: |
| | B 02- 04 + M 13- 20 , |
| | Bereich (C): Sektor: |
| | N 14- 20 + B 02-5, |
| 7 | Bereich (A): Sektor: |
| | ( M 13-16, N 14- 24 + |
| | K 11 -09) x Z 1, 27-0, |
| | Bereich (B): Sektor: |
| | M 13- 20+ O 15- 20 |
| | + O 15- 18, |
| | Bereich (C): Sektor: |
| | E 05- 11 + M 13- 25, |
| 8 | Bereich (A): Sektor: |
| | Z 1,27- 02 ( N 14-11+ N14-20 |
| | + G  7- 14) |
| | Bereich (B): Sektor: |
| | M 13-0 ( M 13-20 + |
| | N 14- 20) |
| | Bereich (C): Sektor: |
| | N 14- 21 ( N 14- 20). |

Diese Koordinaten entsprechen den experimentell aus gesicherter, pathologischer Krankheit ermittelten Texturen und Formen der Kristallisate.

Beispiele der Kristalltexturen und Kristallformen eines gesicherten, pathologischen Krankheitszustandes als Abbildungen für den Analogievergleich mit dem Kristallisat aus Stufe 4 eines Blutes einer nach Stufe 6 zu diagnostizierenden Krankheit.

| Abbildung | Krankheitszustand |
|---|---|
| 9 | Texturen und Formen des Blutes eines 39- jährigen Mannes: chronisch-rheumatische Leukämie: Koordinaten: C 03 -0, C 03- 12, C 03- 18, |
| 10 | Blut einer Frau deren Krankheit zu diagnostizieren ist. Koordinaten: C 03-0, C 03- 12, C 03- 18. Nach Stufe 6 diagnostizierte Krankheit: Akute, chronisch- rheumatische Leukämie. |
| 11 | Blut einer Frau deren Krankheit zu diagnostizieren ist. Koordinaten: C 03- 0, C 03- 12, C 03 - 18. Krankheit: chronisch- rheumatische Leukämie. |

Erläuterungen:

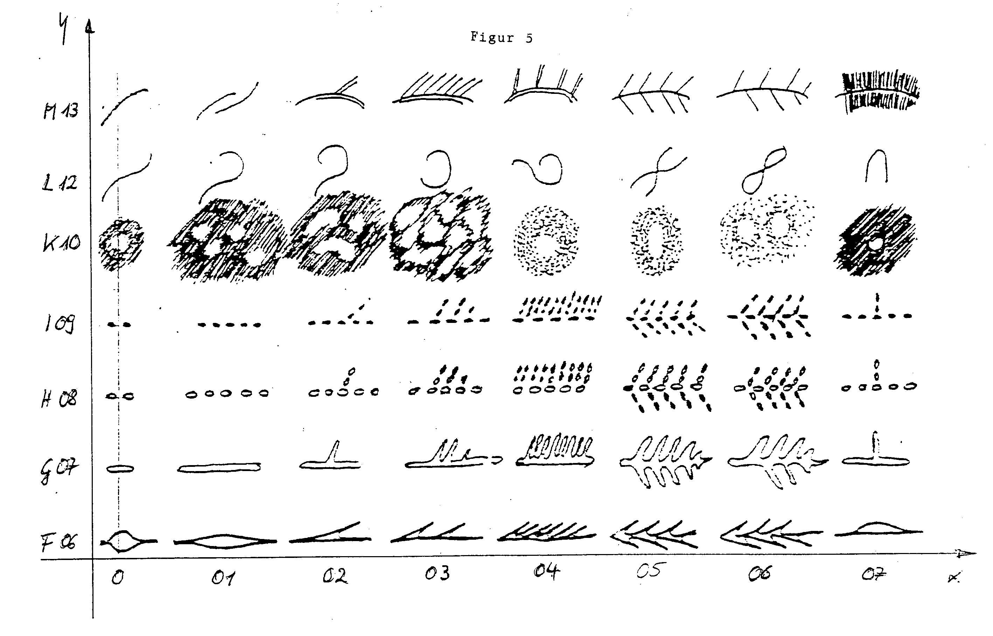

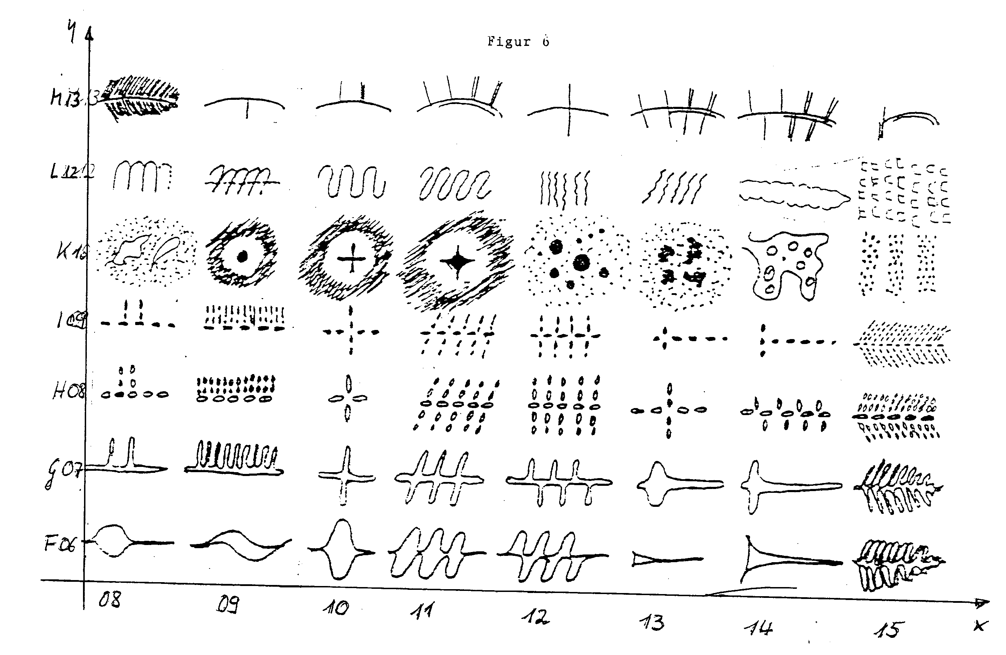

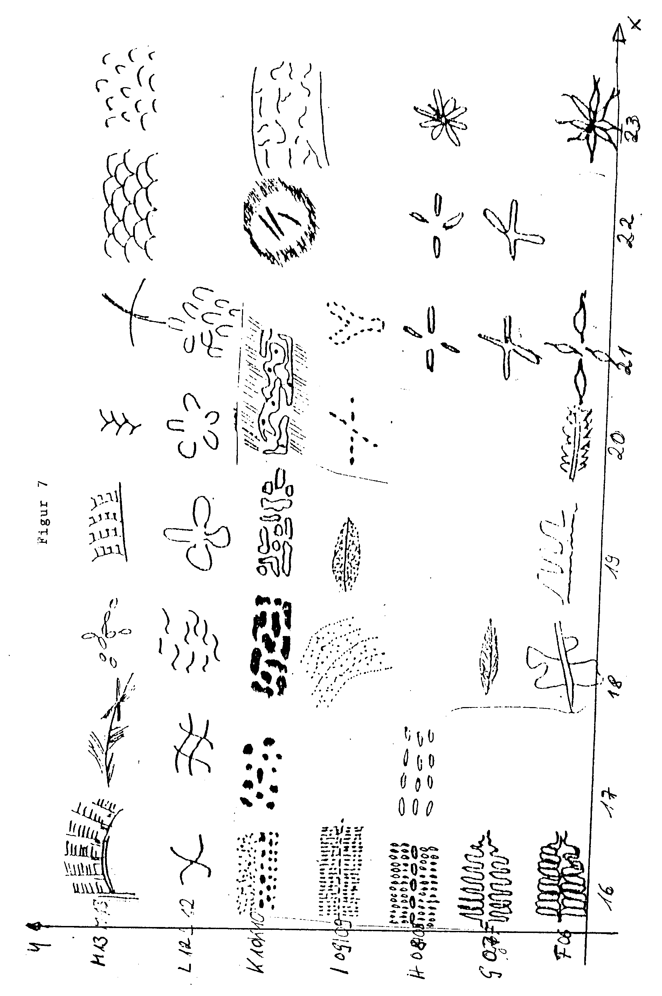

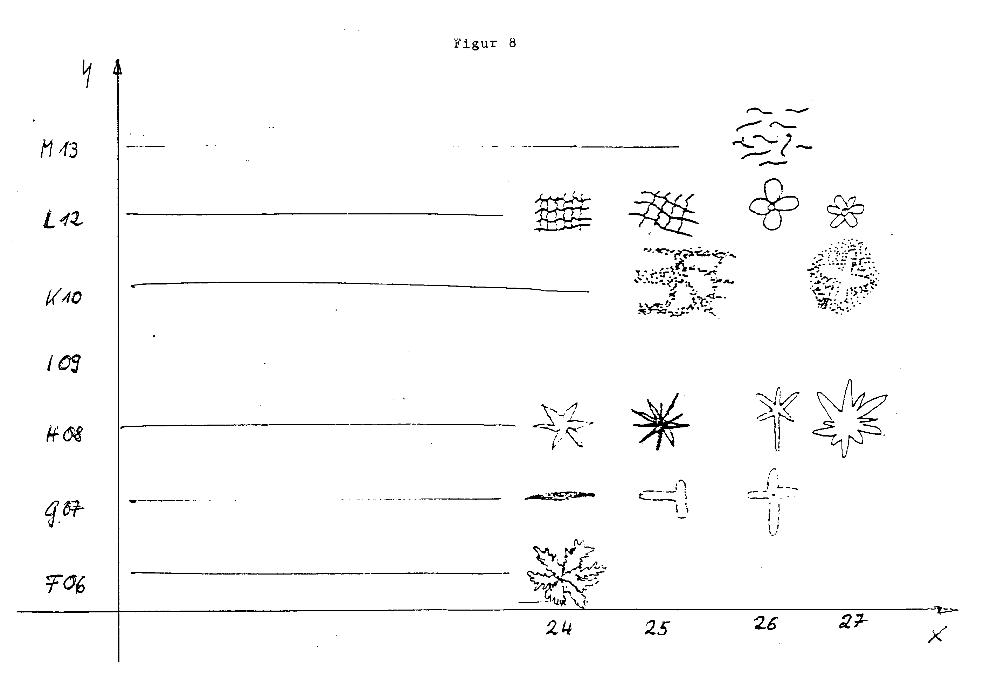

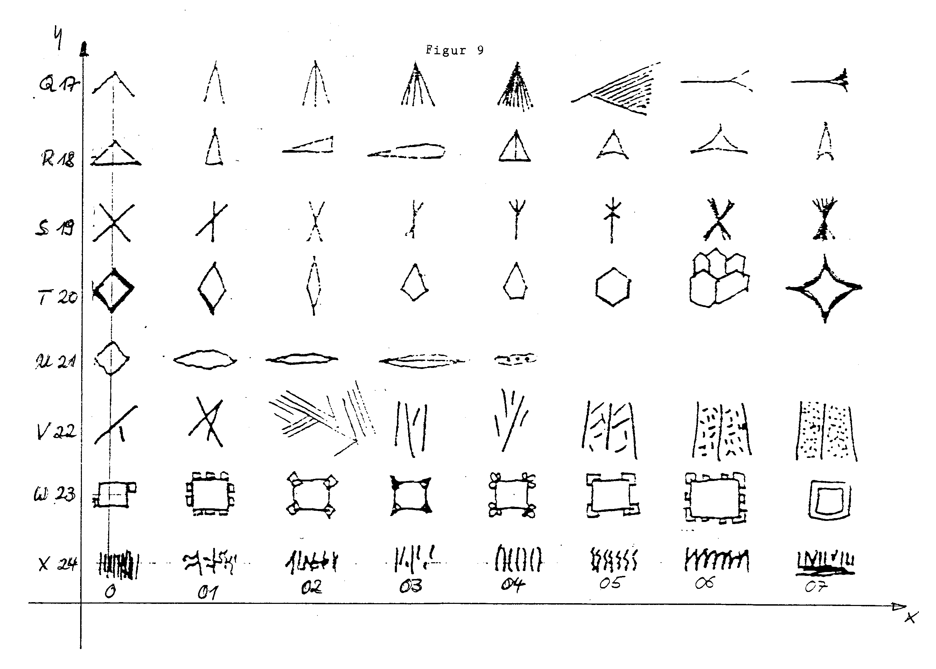

Abbildung 9: Zustand im Stadium 5 der Erkrankung (moribund).Die schalenartigen Schichten zeigen den schubweisen Charakter der Erkrankung im gesamten Körper an.

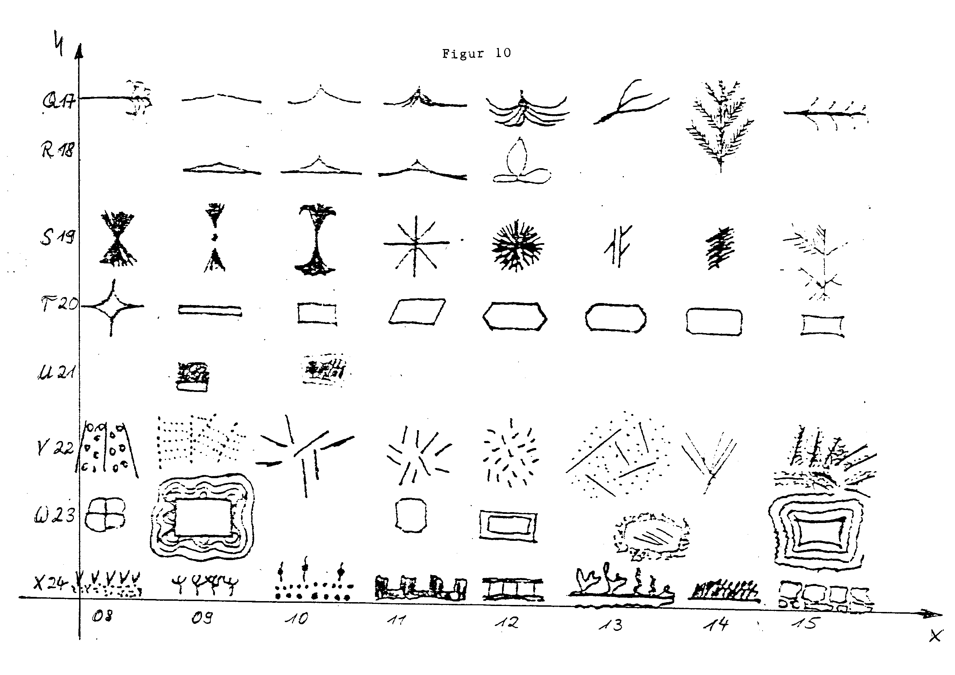

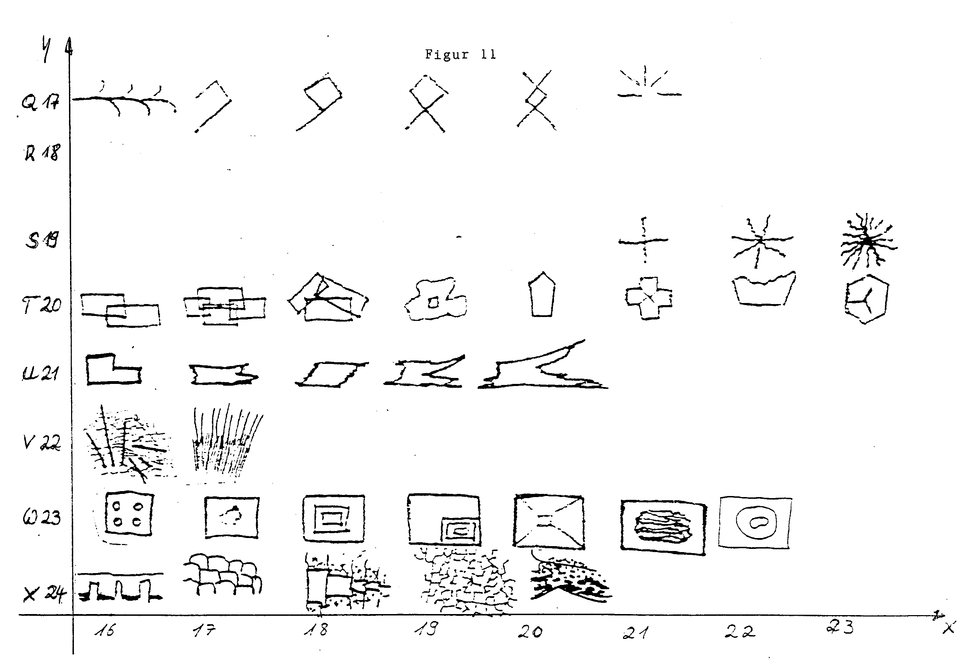

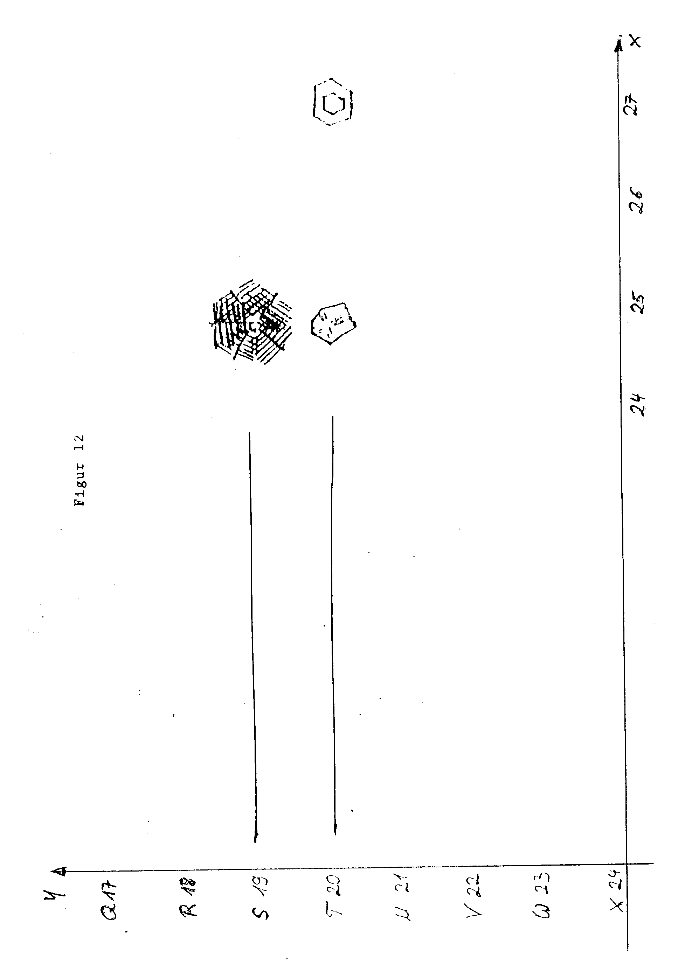

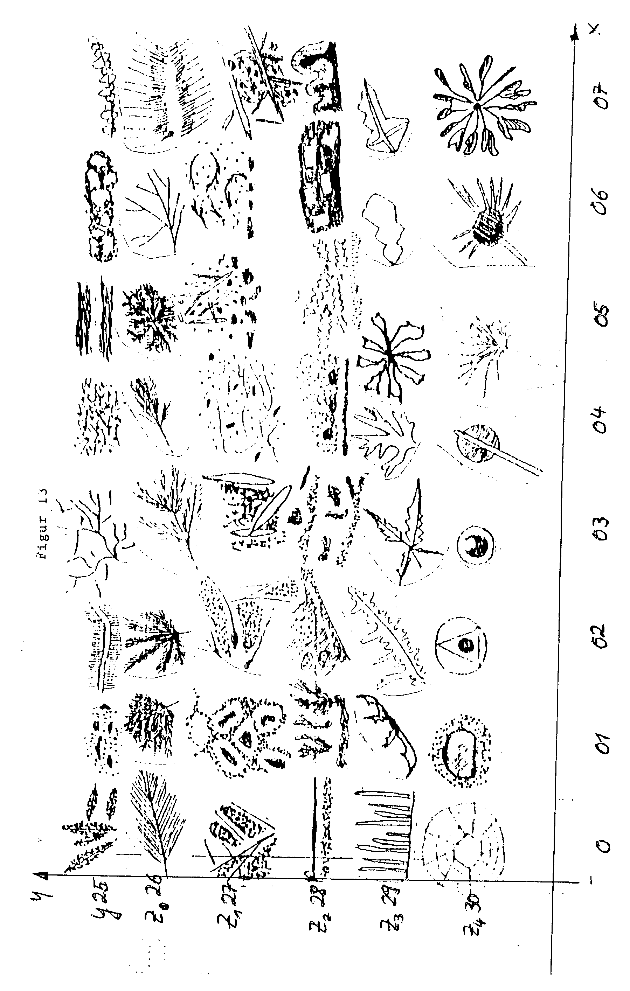

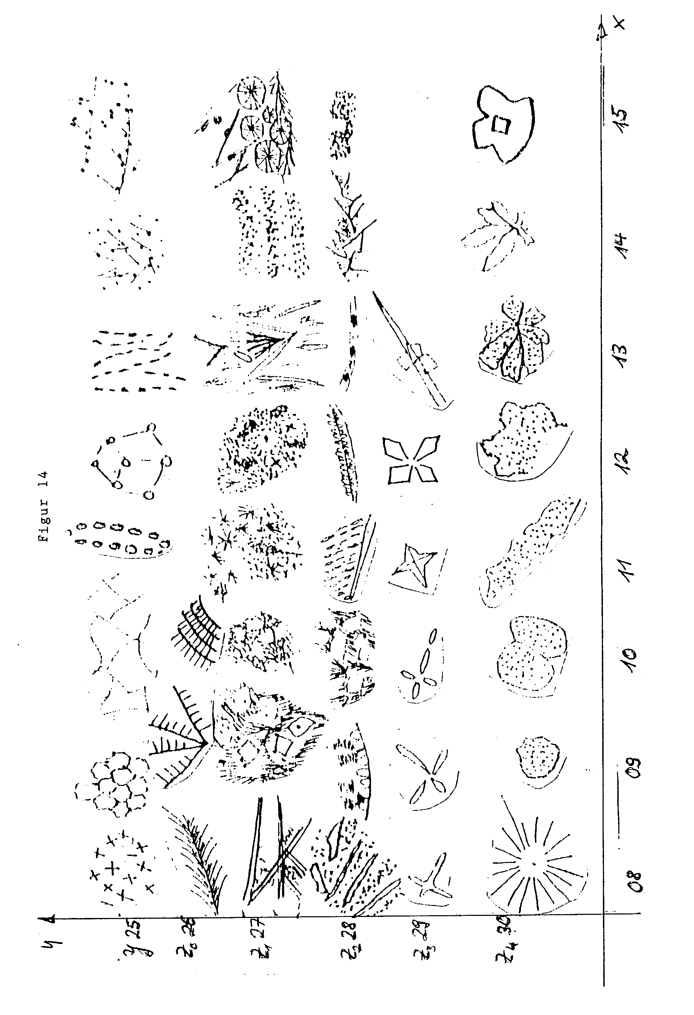

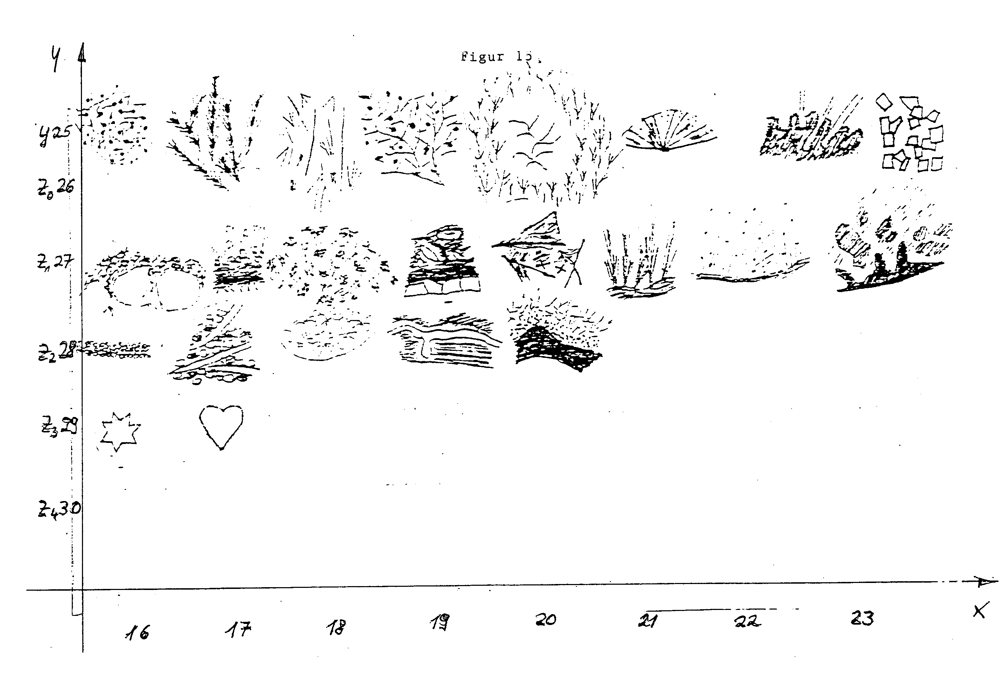

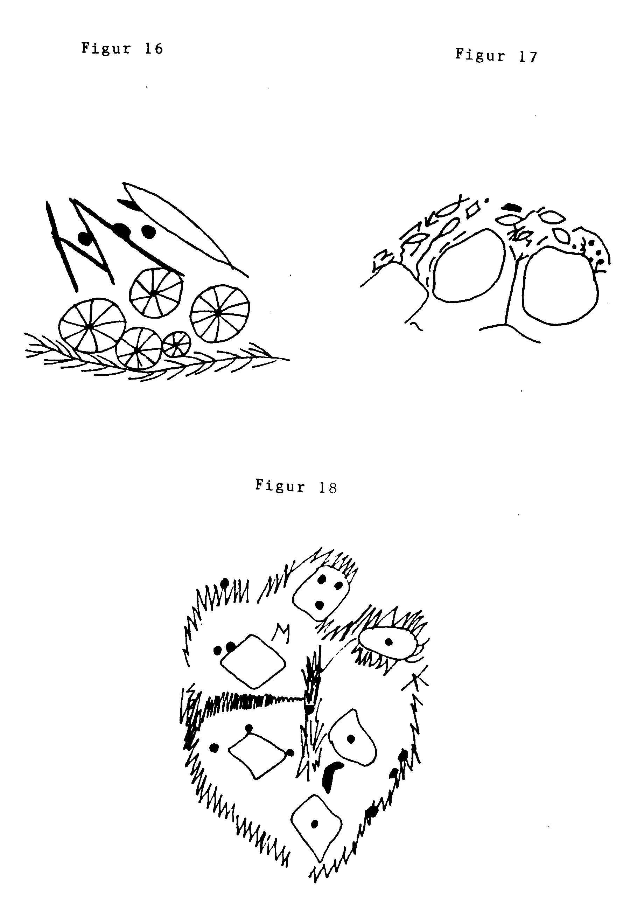

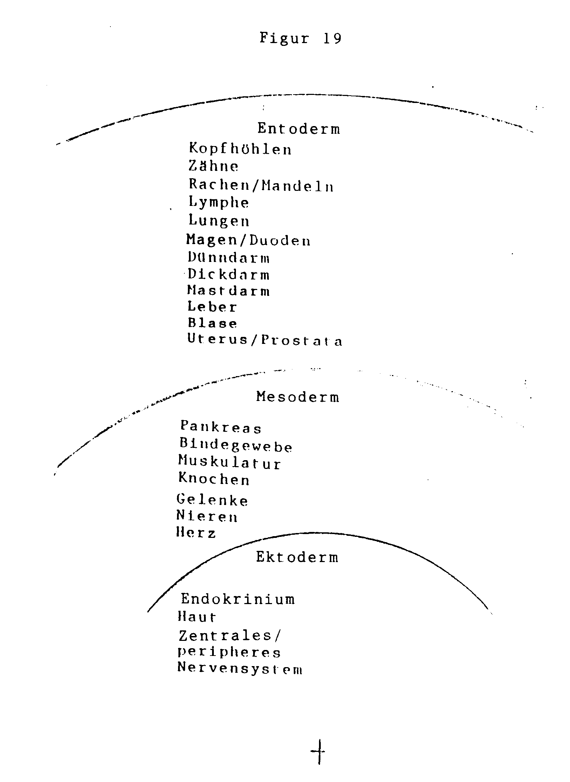

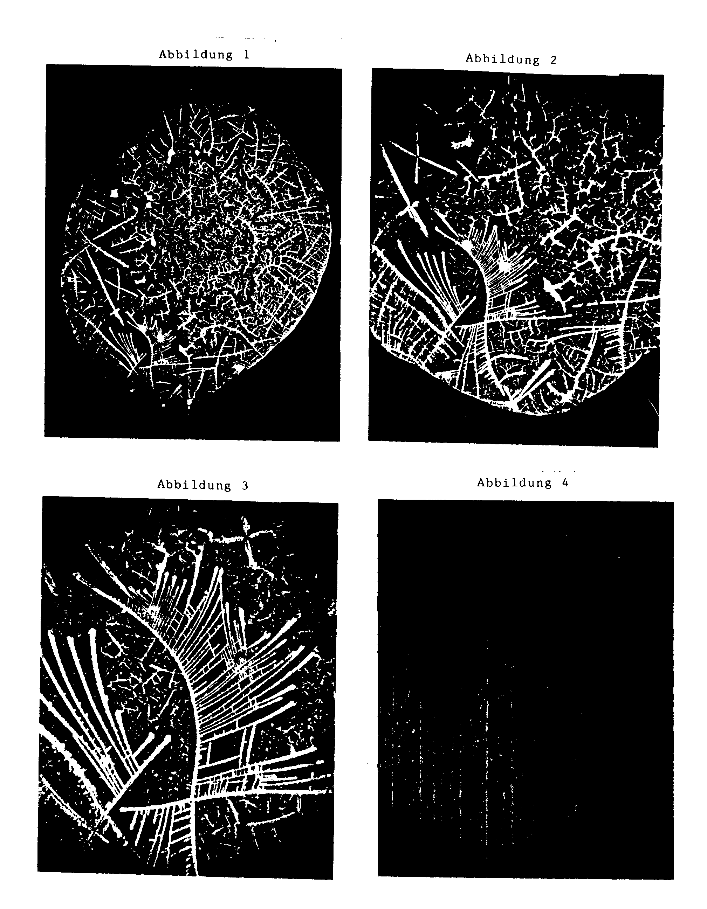

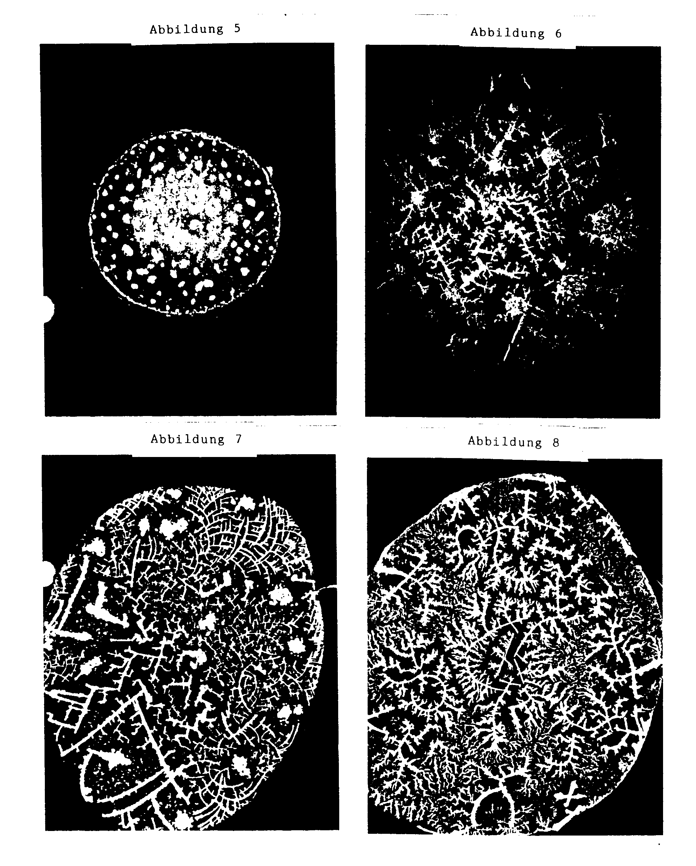

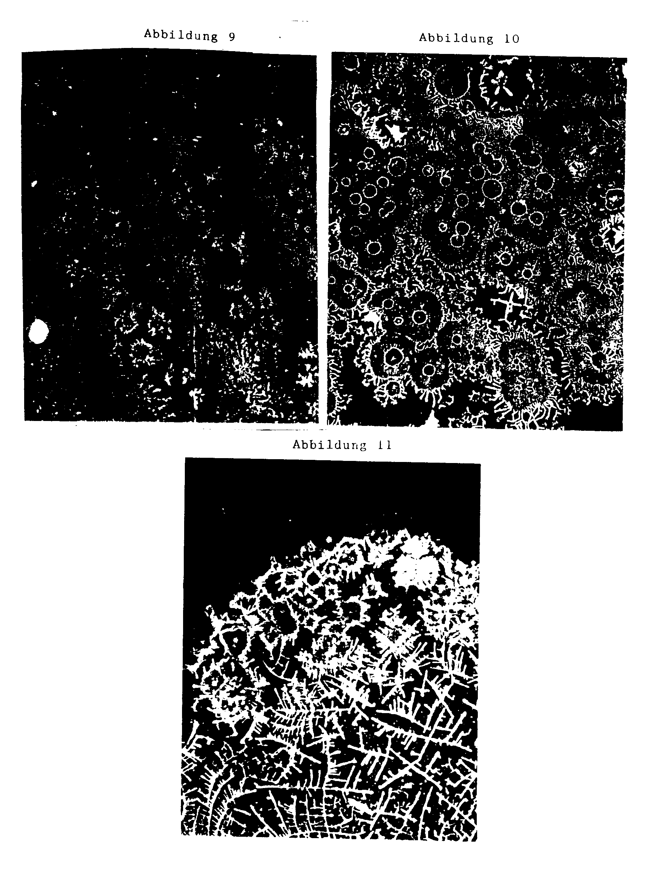

Abbildung 10: Zustand im Stadium 4 mit Metastatisierung der akuten, chronischrheumatischen Leukämie. Nachgewiesen durch die Vergrösserung der Kristallform C 03 - 18.

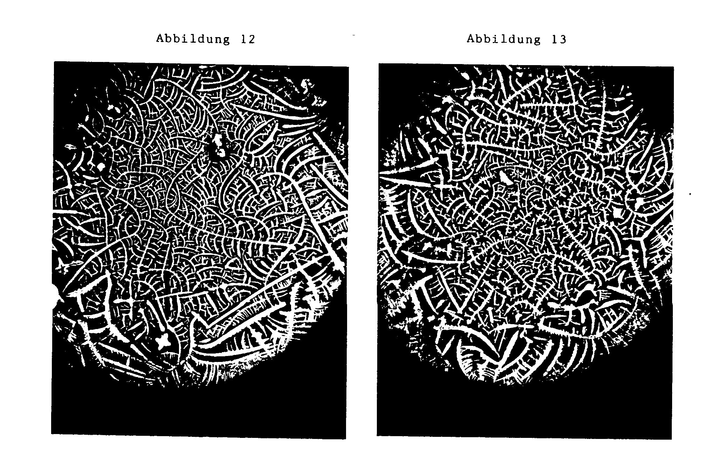

Abbildung 11: Zustand im Stadium 3 mit gleichzeitiger Mykotisierung des Sektors (A) dargestellt an der Korona der Form C 03- 18 und C 03- 0.

Die weiteren Formen auf diesen Abbildungen betreffen nicht das diagnostizierte Krankheitsbild, sondern die individuelle Physiologie bzw. die spezifischen und individuellen, sekundären Folgen der Krankheit.

Die folgenden Abbildungen betreffen die Reproduzierbarkeit der Kristalltexturen und Kristallformen eines Blutbildes nach Aufteilung in 2 Fraktionen.

Diese Abbildungen betreffen das Blut eines Mannes mit Darmentzündung und Gelenkrheumatismus und Pankreatitis.

```
Abbildung      Koordinaten der Kristall-
               texturen:
    12 +       Sektor (A):
    13         Q 17 -15, E 05- 15, M 13- 13,
               Sektor (B):
               E 05- 10, Q 17- 16, T 20- 22,
               Sektor (C): entfällt.
```

Die folgenden Abbildungen betreffen die Reproduzierbarkeit der Kristalltexturen und Kristallformen nach der Aufteilung des Blutes in 4 Fraktionen. Diese Abbildungen betreffen das Blut eines Mannes mit Leberfunktionsschwäche mit beginnender Nierenskerose.

```
Abbildung      Koordinaten der Kristall-
               texturen:
    14 +       Sektor (A): Dominante Form:
    15 +       Q 17 - 05, 0 15- 20,
    16 +       Sektor (B) und
    17         Sektor (C):
               Dominante Form:
               L 12- 0, M 13- 0.
               Beide Formen mit dem
               Abzweigungstyp: N 14- 16.
```

Die Einlagerungen im Sektor (B) demonstrieren ein Nierenkonkrement.

Die Reproduzierbarkeit bei der gleichen Krankheit bei verschiedenen Personen bestätigen beispielsweise die

| Abbildungen | |
| --- | --- |
| 9 + | klinisch gesicherter Zustand, |
| 10 | diagnostizierter Zustand der durch den optischen Analogievergleich der Kristalltexturen und Kristallformen gesichert ist, |
| 11 | desgleichen wie Abbildung 10. |

Die nachstehend aufgeführten Abbildungen demonstrieren beispielsweise die charakteristischen Kristalltexturen für klinisch gesicherte Krankheiten. Diese Abbildungen werden im Analogievergleich nach Stufe 6 verwendet.

| Abbildung | Krankkeit<br>Biologische Koordinaten |
|---|---|
| 18 | Osteomyelofibrose,<br>charakteristische Textur:<br>( N 14 -10 + C 03- 25), |
| 19 | Nephrose bei Aids:<br>charakteristische Textur:<br>M 13- 20 ( E 05- 15), |
| 20 | Mamma Carcinom Stadium 4,<br>charakteristische Textur:<br>Wabenbildung, Typ: K 11-11, |
| 21 | Leberzirrhose,<br>charakteristische Textur:<br>L 12 - ( 01 -04), |
| 22 | offene Tuberkulose,<br>charakteristische Textur:<br>C 03- 02, C 03 - 11, B 02-03, |
| 23 | Syphilis,<br>charakteristische Textur:<br>Y 25 -09 ( K 11-01 + C 03-15), |
| 24 | Myelotische Leukämie,<br>charakteristische Textur:<br>( M 13- 03 + Q 17- 05), V 22-03. |
| 25 | Kleinhirn- Atrophie,<br>charakteristische Textur:<br>( S 19- 12) Y 25- 10. |

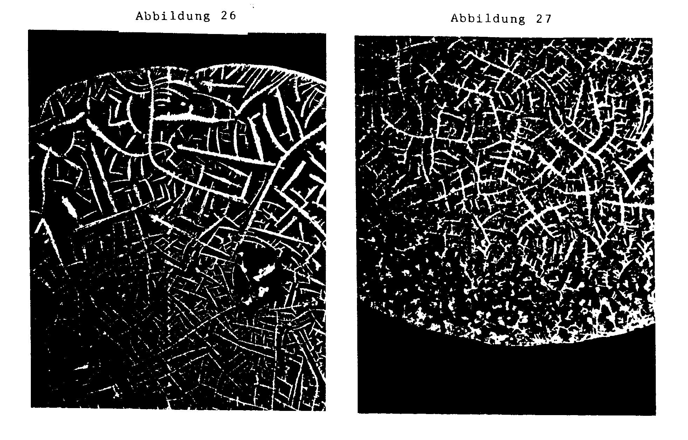

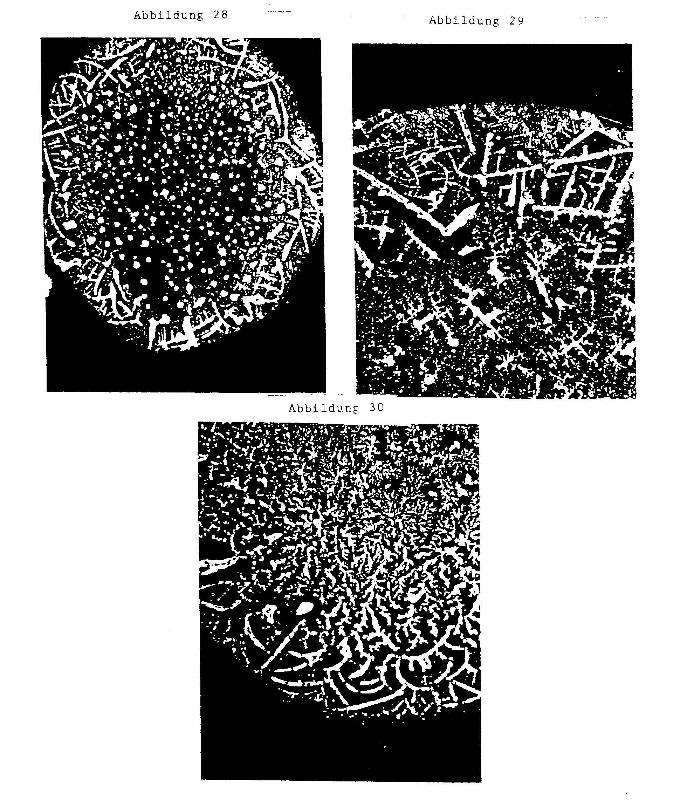

Die Abbildungen 26 und 27 sind aus menschlichem Harn eines Kleinkindes als Ausgangsmaterial nach dem Verfahren der Erfindung hergestellt.

| Abbildung | Krankeit Biologische Koordinaten |
|---|---|
| 26 | Chronische Bronchitis Charakteristische Textur: Sektor A: $Z_1$ 27-05, Sektor B: n(N 14-10) (N 14-11) |
| 27 | akute Nephritis charakteristische Textur: Sektor B: n(N 14-12)(O 15-20) |

Das folgende Ausführungsbeispiel zum Verfahren der Erfindung verwendet Harn als Ausgangsmaterial.Harnanalysen werden herangezogen, wenn die Probenahme von Blut schwierig ist, wie bei Kleinkindern oder bei thrombosege fährdeten Personen oder bei Vorliegen von vernarbten Venen. Venenblut enthält die Stoffwechselprodukte gelöst. Dies gilt ebenso für Harn als Ausscheidungsprodukt und Träger von Stoffwechselprodukten.

Es werden 10 ml Harn aus dem Mittelstrahl eines Kindes mit 30 ml 3-fach destilliertem Wasser vermischt und bei 96°C mit Luftkühlung destilliert. Die Destillation ist beendet, wenn der Rückstand praktisch trocken ist.

In der folgenden Verfahrensstufe wird in gleicher Weise wie mit Blut verfahren und kalziniert. Das Kalzinat wird im Ofen auf 150°C abgekühlt, danach entnommen und im Mörser bis zur feinkörnigen oder weichen Masse verrieben. In der folgenden Verfahrensstufe wird das Destillat aus Stufe 1 mit dem Kalzinat aus Stufe 2 vermischt.

Das Gemisch erhält bei 20°C eine Ruhezeit von 16 h zur Bildung einer homogenen Phase. Dieses wird danach durch mikrofeines Filterpapier filtriert. In der folgenden Verfahrensstufe wird wie mit Blut verfahren zur Ausbildung der Kristalltexturen und Kristallformen. In der folgenden Verfahrensstufe wird wie mit Blut im mikroskopischen Dunkelfeld mit der gleichen, stufenweisen Vergrösserung analysiert und fotooptisch die Abbildung fixiert.

Es werden also mit Harn die gleichen Ergebnisse in den Stufen 5 und 6 erhalten wie mit Blut.

In einer weiteren Verfahrensstufe wird wie mit Blut mit den gleichen Mischungsverhältnissen ein Heilmittel als Autovaxin erzeugt, dem der Handelsname "Homodot" zukommt.

In einer alternativen Durchführung dieser Verfahrensstufe wird dem Harn des Kindes ein Tropfen entnommen, dieser auf Blutagarboden geimpft und bei einer Bruttemperatur von 37°C ein Antivaxin von 2 ml nach 48 h abgezogen. Mit dieser Masse wird wie mit Blut verfahren. Das resultierende Heilmittel erhält den Handelsnamen "Antihomodot". Dieses wird zur spezifischen Simulation chronischer, toxischer und parasitogener Herde im Körper dieses Kindes verwendet.

Die Ausbildung der Kristalltexturen oder Kristallformen erfolgt mit tierischem Blut oder Harn in analoger Weise.

Die folgenden Abbildungen sind aus tierischem Blut von Pferden als Ausgangsmaterial nach dem Verfahren der Erfindung hergestellt.

| Abbildung | Krankheit |
|---|---|
| | Biologische Koordinaten |
| 28 | |
| | motorische Lähmung |
| | Charakteristische Textur: |
| | Sektor A: |
| | M13-11, M13-16; |
| | Sektor B und C: |
| | K11-12. |
| 29 | Niereninfektion |
| | Charakteristische Textur: |
| | Sektor A: |
| | N14-24, $Z_1$27-01. |
| | Sektor B: |
| | F06-23, $Z_1$27-11 |
| 30 | Nephrose und Hepatose |
| | Charakteristische Textur: |
| | Sektor A: |
| | M13-20, D04-07; |
| | Sektor B. |
| | (E05-12)(M13-20) |
| | Sektor C: |
| | (N14-15)(M13-20). |

Die Abbildung 31 demonstriert in den konzentrischen Bereichen und zwar in dem entodermalen Bereich (A),in dem mesodermalen Bereich (B) und in dem ektodermalen Bereich (C) die definierte Reihenfolge der Organe und Organzonen von aussen nach innen.

Diese Feststellung der Lokalisierung erfolgt durch optische Auswertung der Kristalltexturen und Kristallformen, die nach dem Verfahren der Erfindung erzeugt sind.Diese Texturen und Formen sind aus dem Blut von klinisch gesicherten Krankheitsbildern von Personen erzeugt.

Durch die Zuordnung ist es überraschend möglich, die Reihenfolge und den Ort zu bestimmen und zu definieren, an denen diese Erkrankungen aus den für sie charakteristischen Figuren der Texturen und Formen zu erkennen sind.

Die Reproduzierbarkeit der Zuordnung ergibt sich dadurch, dass das Blut mehrerer Personen mit der gleichen, gesicherten Krankheit analysiert wird.

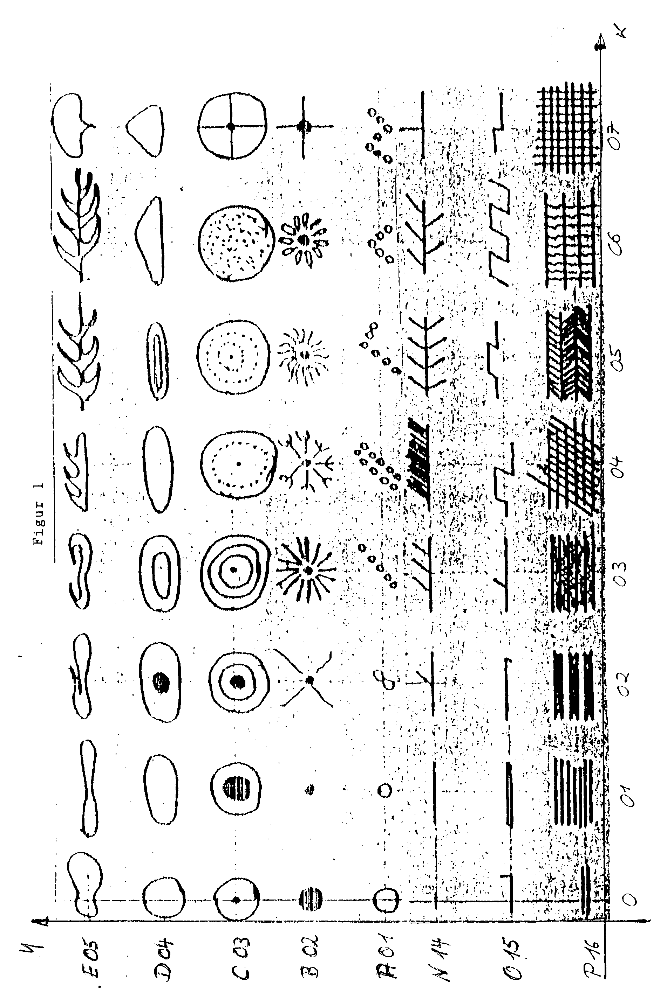

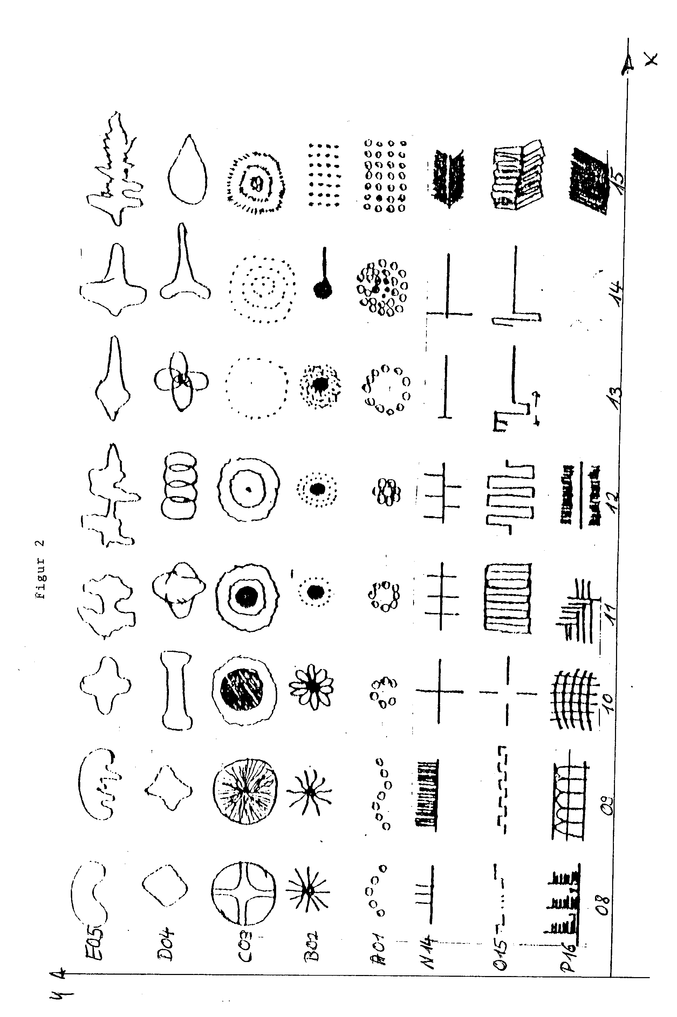

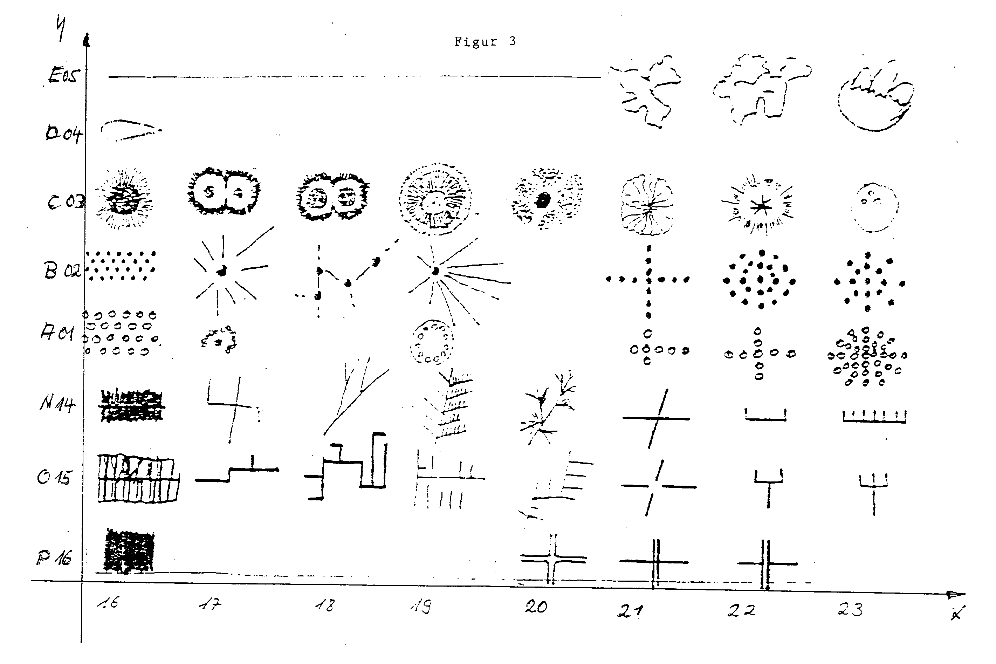

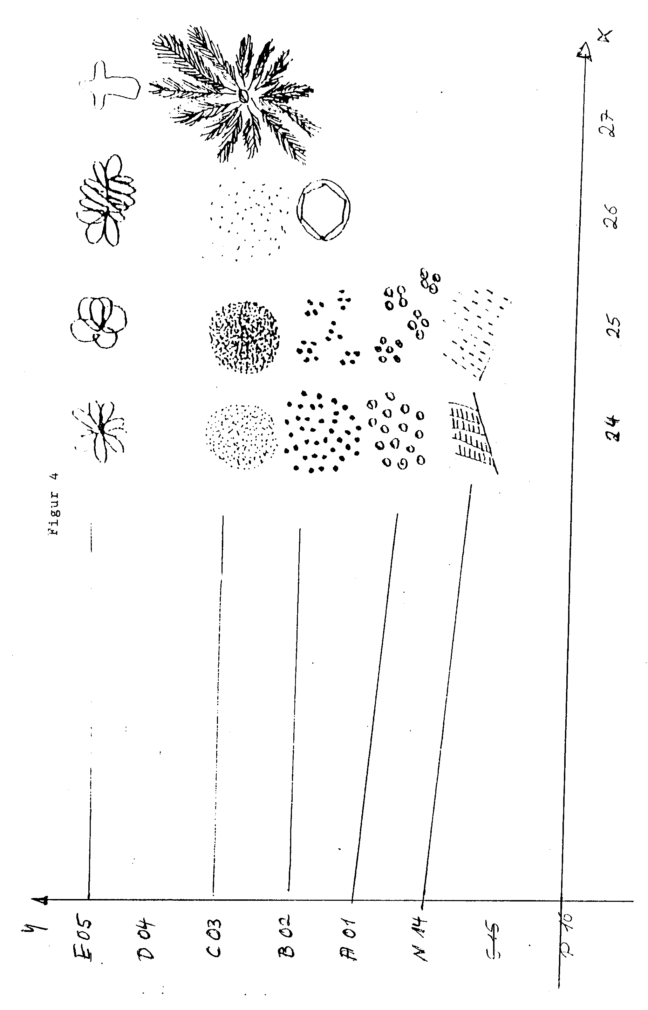

Diese Funktionskreise (A), (B) (C) sind auch in Figur 4 dargestellt.

Diese Figuren zeigen in gegenüber der Natur reziproker Darstellung diese Bereiche in den konzentrischen Zonen. Diese zeigen in diesen Bereichen die Topologie der Organe und Organzonen.

Die im Anspruch 6 beanspruchten Koordinaten sind auch in den folgenden Figuren dargestellt:

| Figur: | Koordinate: |
|--------|-------------|
| 13 | $y = Y\ 25;\ x = (01 - 07)$<br>$y = Z_0\ 26;\ x = (01 - 07)$<br>$y = Z_1\ 27;\ x = (01 - 07)$<br>$y = Z_2\ 28;\ x = (01 - 07)$<br>$y = Z_3\ 29;\ x = (01 - 07)$<br>$y = z_4\ 30;\ x = (01 - 07)$ |
| 14 | $y = Y\ 25;\ x = (08 - 15)$<br>$y = Z_0\ 26;\ x = (08 - 10)$<br>$y = Z_1\ 27;\ x = (08 - 15)$<br>$y = Z_2\ 28;\ x = (08 - 15)$<br>$y = Z_3\ 29;\ x = (01 - 07)$<br>$y = Z_4\ 30;\ x = (08 - 15)$ |
| 15 | $y = Y\ 25;\ x = (16 - 23)$<br>$y = Z_1\ 27;\ x = (16 - 23)$<br>$y = Z2\ 28;\ x = (16 - 20)$<br>$y = Z_3\ 29;\ x = (16 - 17)$ |

Die weiter in Anspruch 6 aufgeführten vorzugsweisen Koordinaten sind in den folgenden Abbildungen dargestellt:

Fig.16     $y = Z_1\ 27;\ x = 15$:
       $(y = C\ 03;\ x = 09)$, $(y = N\ 14;\ x = 18)$, $(y = V\ 22;\ x = 06)$

Fig.17     $y = Z_1\ 27;\ x = 16$:
       $n(y = D\ 04;\ x = 0)$, $(y = Q\ 17;\ x = 06$, $y = A\ 01;\ x = 24)$

Fig.18     $y = Z_1\ 27;\ x = 09$:
       $(y = K\ 11;\ x = 03)$, $(y = T\ 20;\ x = 0$, $y = W\ 23;\ x = 0$, $y = W\ 23;\ x = 17$, $y = C\ 03;\ x = 34$, $y = C\ 03;\ x = 0)$

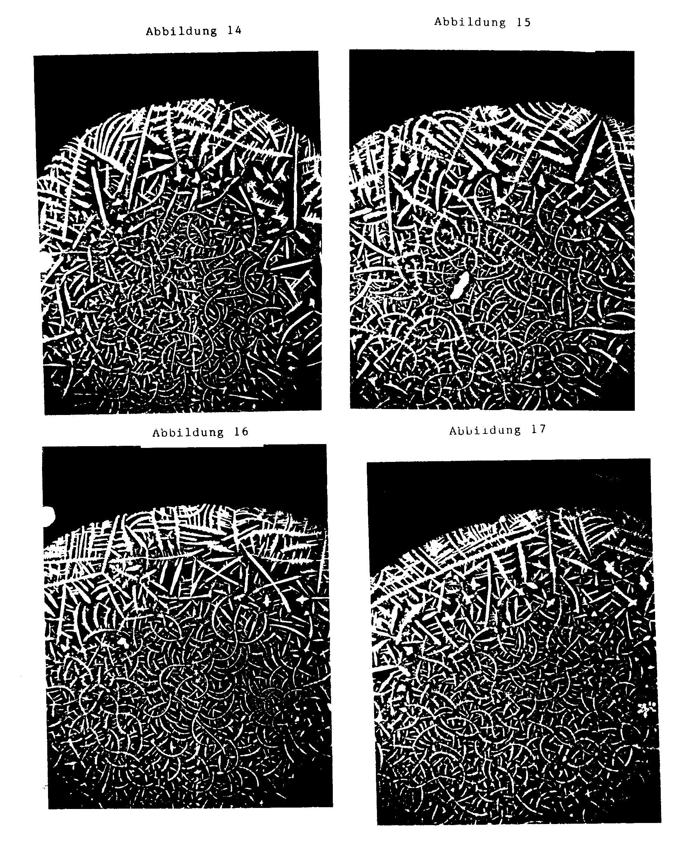

Die Figuren 16 bis 18 stellen geometrische Figuren als Koordinaten dar, abgeleitet aus Kristalltexturen und Kristallformen von Mutterformen und von Tochterformen durch Hinzufügen, Verknüpfen und Überlagern.

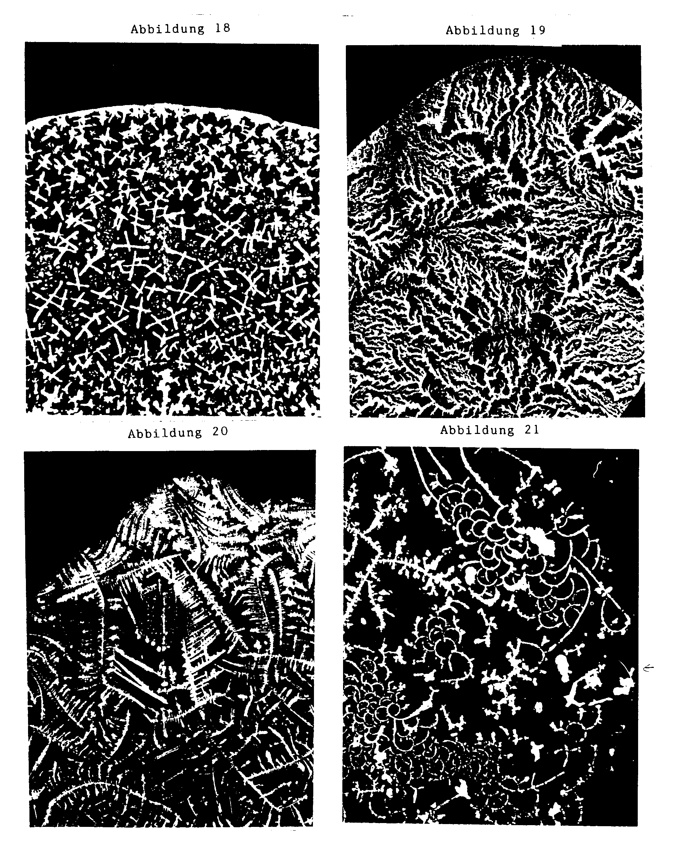

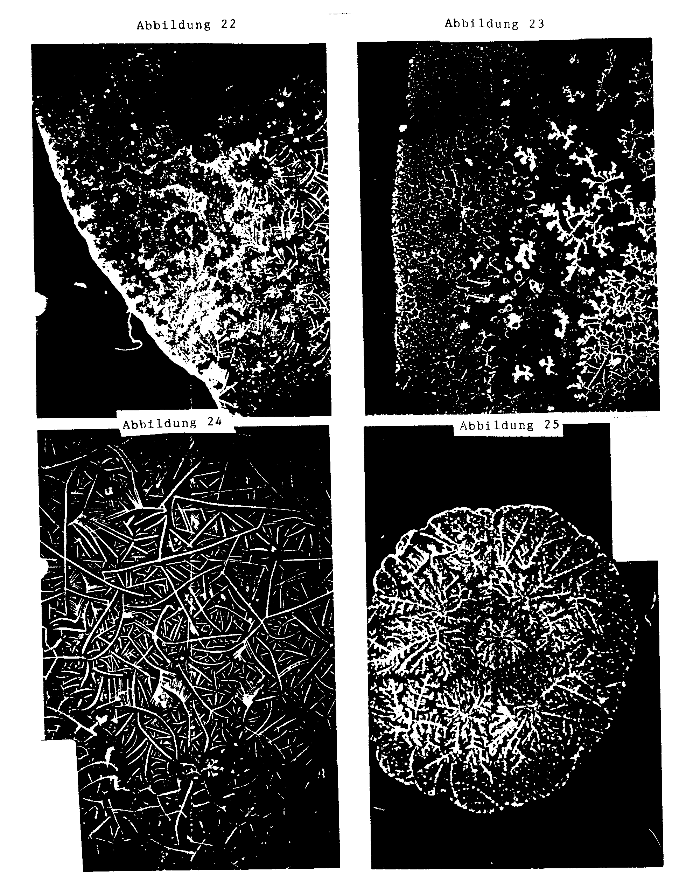

Die Figur 19 stellt innerhalb der konzentrischen Kreise die Ablesefolge von aussen nach innen der den Kristalltexturen und Kristallformen zugeordneten Organen und Organzonen dar.

Die Durchführung des Verfahrens der Erfindung zur Frühdiagnose und zur Herstellung des Heilmittels ist ökologisch positiv, da keine toxisch wirkende Substanzen, wie Chemikalien, benötigt werden und somit auch keine Rückstände daraus entstehen.

Das Verfahren der Erfindung hat durch die Möglichkeit der Früherkennung von Krankheitszuständen bei Mensch und Tier erhebliche Bedeutung für die medizinische Wissenschaft und Praxis. Dieses ermöglicht durch rechtzeitige Therapierung von Krankheiten die Kostenerniedrigung gegenüber denen bei fortgeschrittenen Krankheitszuständen.

Dem Verfahren der Erfindung kommt somit auch eine erhebliche, volkswirtschaftliche Bedeutung zu.

Nach dem Stand der medizinischen Behandlung von Krankheiten sind zu deren Feststellung eine Vielzahl von teilweise aufwendigen Diagnosemethoden erforderlich.

Dagegen gestattet das Verfahren der Erfindung mit einer technisch in einfacher Weise durchzuführenden Arbeitsweise mit Geräten mit geringeren Investierungskosten rasch und sicher die Frühdiagnose durchzuführen und gegebenfalls die erkannten Krankheiten mit,aus dem gleichen Blut hergestellten, Heilmitteln zu therapieren.

Das Verfahren der Erfindung gestattet also, viele Parameter für eine ganzheitliche qualitative Darstellung mit einer Apparatur reproduzierbar zu erfassen.

## Patentansprüche

1. Verfahren zur kristallmorphologischen Blut- und Harnanalyse zur Frühdiagnose und zur Herstellung von Arzneimitteln unter Erzeugung eines Destillates und eines Kalzinates aus menschlichem Blut oder Ausscheidungen unter Vermischung zu einer Dispersion, unter Bildung von Kristallisaten und Analogievergleich mit typbildenden Kristallbildern von Kristalltexturen und Kristallformen, gekennzeichnet durch folgende Verfahrensstufen:

    1) Vermischen des menschlichen Blutes oder Harns oder des tierischen Blutes oder Harns von Warmblütern mit mindestens 3-fach destilliertem Wasser im Volumenverhältnis von 1:2.5 bis 3.5 vorzugsweise von 1: 2.7 bis 3.2 und drucklose Destillation bei Siedetemperatur bis zur Bildung eines Destillates und eines praktisch trockenen Blutkuchens oder Harnsedimentes.

2) Kalzination des trockenen Blutkuchens oder Harnsedimentes linear ansteigend bis auf 600°C bei bis maximal 1 % Streuung in einer Aufheizzeit von 60 bis 70 min vorzugsweise von 65 min bei konstanter Heizleistung, langsame Abkühlung des Kalzinates bis auf etwa 150°C in etwa 2 bis 4 h, Pulverisierung des abgekühlten Kalzinates.

3) Vermischen des Destillates aus Stufe 1 und des Kalzinates aus Stufe 2. Einschalten einer Ruhezeit von etwa 8 bis 16 h zur Bildung einer homogenen Phase. Filtration der flüssigen Mischung.

4) Aufbringen der gefilterten Lösung mit 19.9 bis 20.1 °C, vorzugsweise mit 20.0°C bei einer relativen Luftfeuchtigkeit von etwa 40 bis 60%, auf gläserne, mikroskopische Objektträger in Form von 2 Tropfen mit einem Durchmesser von 6 bis 10 mm, vorzugsweise von 8 mm. Verdunsten des Wasser bei 19.9 bis 20.1 °C unter Ausbildung von praktisch trockenen Kristalltexturen mit Kristallformen und deren Zuordnung zu definierten menschlichen oder tierischen Organen oder physiologischen Funktionskreisen oder Keimblattsektoren, derart, dass dem endodermalen Bereich (A) eine bevorzugte Teilfläche in radialer Richtung im äusseren, konzentrischen Ringraum und dem mesodermalen Bereich (B) eine bevorzugte Teilfläche in radialer Richtung im mittleren, konzentrischen Ringraum und dem ektodermalen Bereich (C) der zentrale Ringraum zugeordnet ist.

5) mikroskopische Ermittlung der Kristalltexturen und Kristallformen im Dunkelfeld mit

| Vergrösserungen | zur Darstellung: |
|---|---|
| 10 bis 15-fach | der Sektoren im Gesamtbild, |
| 20 bis 50-fach | der einzelnen Sektoren (A), oder (B), oder (C), nach Stufe 4, |
| 60 bis 160-fach | der kompakten, flächenhaften und/oder räumlichen Dendriten als Texturen |
| 170 bis 800-fach | der Dendritbilder mit morphologischen, geometrischen, räumlichen Grundformen zur Identifizierung der kristallbildenden Grundformen. |

6) mikroskopischer und fotooptischer Analogievergleich der in Stufe 5 definierten Kristalltexturen und Kristallformen mit solchen eines gesicherten, pathologischen Zustandes aus Vergleichsserien als Standardtypen oder mit den Koordinaten von Mutter-und Tochterformen zur Diagnose des Gesundheits-, oder des Krankheitszustandes von Mensch oder von Wirbel- und Säugetieren.

2. Verfahren nach Anspruch 1, dadurch gekennzeichnet, dass in Stufe 1 eine Menge des menschlichen oder des tierischen Blutes oder des Harns von 3 bis 15 ml, vorzugsweise von 7 bis 10 ml, eingesetzt wird.

3. Verfahren nach den Ansprüchen 1 und 2, dadurch gekennzeichnet, dass die Kalzination in Stufe 2 3-stufig bei Temperaturen von 200°, 400°, 600 °C in der Weise erfolgt, dass erst linear auf 200°C erhitzt und bei dieser Temperatur 5 bis 10 min konstant gehalten wird, und diese Arbeitsweise bei 400°C und bei 600°C wiederholt wird, und danach die Abkühlung gemäss Stufe 2 erfolgt.

4. Verfahren nach den Ansprüchen 1 bis 3, dadurch gekennzeichnet, daß die Kristalltexturen und Kristallformen folgende MUTTERFORMEN als geometrische, morphologische Figuren aufweisen:

| Abszisse | Ordinate | |
|---|---|---|
| x = 0; | y = A 01: | Leerer Kreis mit maximalem Durchmesser von 0,2 mm auf dem Objektträger. |
| x = 0; | y = B 02: | Gefüllter Kreis von 0 bis maximal 2 mm Durchmesser auf dem Objektträger, |
| x = 0; | y = C 03: | Leerer Kreis mit kleiner, gefüllter Kreisfläche im Inneren mit beliebigem Durchmesser, |
| x = 0; | y = D 04: | Leerer Kreis mit Durchmesser größer 0,2 mm auf dem Objektträger, |
| x = 0; | y = E 05: | Tropfenähnliche Form mit Rundung an beiden Längsseiten, |
| x = 0; | y = F 06: | Oval gestreckte Form mit Rundung an beiden Längsseiten, |
| x = 0; | y = G 07: | Zusammengedrückter, leerer Kreis mit parallelen Längsseiten und runden Ecken, |
| x = 0; | y = H 08: | Zwei zusammengedrückte, leere Kreise |
| x = 0; | y = I 09: | Zwei kleine, leicht zusammengedrückte, gefüllte Kreise, |
| x = 0; | y = K 11: | Binnenkreis in einer diffusen oder geometrisch geordneten Fläche, |
| x = 0; | y = L 12: | Sinusförmige Linie, |
| x = 0; | y = M 13: | Kreisförmige Grenzlinie eines Segments, |
| x = 0; | y = N 14: | Einfache Strichform, |
| x = 0; | y = O 15: | Striche mit Abwinkelungen, |
| x = 0; | y = P 16: | Scharen, parallele Linienformen |
| x = 0; | y = Q 17: | Winkel- und winkelige Formen, |
| x = 0; | y = R 18: | Dreiecks- und dreieckige Formen, |
| x = 0; | y = S 19: | Kreuzungs- und sich kreuzende Formen, |
| x = 0; | y = T 20: | Vierecks- und viereckige Formen, |
| x = 0; | y = U 21: | Gebrochene Vierecksformen, |
| x = 0; | y = V 22: | Gößere Linien mit an- und ungebundener, kleinerer, gewinkelter Linie. |
| x = 0; | y = W 23: | Größere Rechteckformen mit kleinerer, eckiger Anlagerung an den Außenseiten, |
| x = 0; | Y = X 24: | Querläufige Bänderform. |

5. Verfahren nach den Ansprüchen 1 bis 4, dadurch gekennzeichnet, daß die Kristalltexturen und Kristallformen der von den Mutterformen abgeleiteten Tochterformen folgende geometrische und morphologische Figuren aufweisen:

| Mutterformen (1): | Abszisse x = 0; | Ordinate y = n |
|---|---|---|
| Tochterformen (2): | Abszisse x = (01 - m); | Ordinate Y = n |

| Koordinaten | Figürliche Darstellung |
|---|---|
| (1) y = A 01 | Kreisförmige Formen mit ungefüllter |
| (2) x = 01 - 17, | Innenfläche, deren Aufreihungen, |
| 19, 21 - 26 | Akkumulationen, geordneter und |
| | ungeordneter Rasterbildung, |
| (1) y = B 02 | Kreisförmige Formen mit gefüllter |
| (2) x = 01 - 19, 21 - 26 | Innenfläche, deren haufenförmige, |
| | regelmäßige oder unregelmäßige |
| | Rasterbildungen, sowie deren radiale, |
| | zentrifugale, freie oder vernetzte |
| | Anlagerungen und Ringbildungen, |
| (1) y = C 03 | Kreisförmige Formen mit dichterer |

16

```
(2) x = 01 - 27          punkt-, flächen-, haufenförmiger,
                         gefüllter Innenfläche oder mit radialen
                         oder kreuzenden oder zentrifugalen
                         Einzel-, Misch- oder Binnenformen,
(1) y = D 04             Ovale und geometrische Formen mit
(2) x = 01 - 16          überlagerter Kontur,
(1) y = E 05             Ovale Streckformen mit Abzweigungen,
(2) x = 01 - 15, 21 -27  Einbuchtungen, radialen oder flächenden
                         Anlagerungen,
(1) y = F 06             Runde, längliche, gestreckte oder
(2) x = 01 - 16, 18 - 21 verzweigte Formen mit spitzen Enden
        23 - 24          und leerer Innenfläche,
(1) y = H 08             Reihen, Fächer, Verzweigungen und
(2) x = 01 - 17, 20 - 27 geometrische Rasterbildungen aus kleinen,
                         leeren, gedrückten Kreisen,
(1) y = I 09             Reihen aus kleinen, leeren, gedrückten
(2) x = 01 - 19, 21 - 22 Kreisen mit Verzweigungen, Häufungen,
                         aus kleinen, gefüllten Kreisen,
(1) y = K 11             Unregelmäßige, verteilte, negative
(2) x = 01 - 23, 25, 27  Kreise in geometrisch geordneten oder
                         ungeordneten Flächen, frei oder mit
                         geometrischen Formen gefüllt, wie deren
                         positive Abbildungen,
(1) y = L 12             Sinusförmige, einfache, verknüpfte,
(2) x = 01 - 21, 24 - 27 kreuzende, haufenförmige Formen,
                         geometrische Raster bildend,
(1) y = M 13             Gebogene Linien mit Kreuzungen
(2) x = 01 - 23, 26      kamm- und leiterförmige Formen,
                         geometrische Raster bildend,
(1) y = N 14             Einfache Linienformen mit ein- und
(2) x = 01 - 25          beidseitigen, wechsel- oder gegen-
                         ständigen Abzweigungen,
(1) y = O 15             Einfache und geknickte Linienformen
(2) x = 01 - 25          mit ein- und beidseitigen, wechsel-
                         oder gegenständigen Abzweigungen,
(1) y = P 16             Scharen, doppelte oder mehrfache, meist
(2) x = 01 - 12, 15 - 16 parallele Linienformen, sich kreuzende,
        20 -22           meiste parallele Linienformen,
(1) y = Q 17             Winkelige Linienformen mit büschelartigen
(2) x = 01 -21           Abzweigungen und/oder mit Bogenbildungen
                         in den Winkelschenkeln,
                         sowie deren Kreuzungen,
(1) y = R 18             Dreieckige Formen,
(2) x = 01 - 07, 09 - 12
(1) y = S 19             Einfache Kreuzungsformen, sowie deren
(2) x = 01 - 15, 21 - 23, 25 Bündelungen zu geometrischen Formen
(1) y = T 20             Vier- und mehreckige Formen und deren
(2) x = 01 - 23, 25, 27  Agglomerate,
(1) y = U 21             Vier- und mehreckige Formen mit unregel-
(2) x = 01 - 04, 09 - 10 mäßigen, teilweise deformierten Konturen,
        16 - 20
(1) y = V 22             Langgestreckte Linienformen mit freier
(2) x = 01 - 17          oder kreuzender Anordnung zu kürzeren,
                         langgestreckten Formen oder
                         zu punktförmigen Formen,
(1) y = W 23             Viereckige Formen mit geometrischen
(2) x = 01 - 09, 11 - 22 Anlagerungen an den Außenseiten und
                         geometrischen Einlagerungen in die
                         Innenflächen,
(1) y = X 24             Bänderformen aus zur Bänderlaufrichtung
(2) x = 01 - 20          quer oder winkelig liegende Elemente und
```

deren Verbindungen oder aus punktförmigen
Anreihungen oder aus periodischen, freien
oder geometrischen Formen,

17

EP 0 307 566 B1

sowie die Formen nach den Koordinaten:

| | |
|---|---|
| y = Y 25 | x = 01 - 23 |
| y = $Z_0$ 26 | x = 01 - 10 |
| y = $Z_1$ 27 | x = 01 - 23 |
| y = $Z_2$ 28 | x = 01 - 20 |
| y = $Z_3$ 29 | x = 01 - 13, 16, 17 |
| y = $Z_4$ 30 | x = 01 - 15 |

6. Verfahren nach den Ansprüchen 1 bis 5, dadurch gekennzeichnet, daß die Kristalltexturen und Kristallformen komplexe und/oder von den Mutterformen und von den Tochterformen abgeleitete geometrische und morphologische Figuren als Koordinaten aufweisen, wobei der Ausdruck vor einer Klammer die übergeordnete, figurenbildende Form und der Ausdruck in einer Klammer eine untergeordnete Teilform darstellen, vorzugsweise mit den Koordinaten:

y = $Z_1$ 27; x = 15:
 (y = C 03; x = 09), (y = N 14; x = 18), (y = V 22; x = 06)
y = $Z_1$ 27; x = 16:
 n(y = D 04; x = 0), (y = Q 17; x = 06, y = A 01; x = 24)
y = $Z_1$ 27; x = 09:
 (y = K 11; x = 03), (y = T 20; x = 0, y = W 23; x = 0,
 y = W 23; x = 17, y = C 03; x = 34, y = C 03; x = 0)
y = Y 25; x = (01 - 07)
y = $Z_0$ 26; x = (01 - 07)
y = $Z_1$ 27: x = (01 - 07)
y = $Z_2$ 28; x = (01 - 07)
y = $Z_3$ 29; x = (01 - 07)
y = $Z_4$ 30; x = (01 - 07)
y = Y 25; x = (08 - 15)
y = $Z_0$ 26; x = (08 - 10)
y = $Z_1$ 27; x = (08 - 15)
y = $Z_2$ 28; x = (08 - 15)
y = $Z_3$ 29; x = (01 - 07)
y = $Z_4$ 30; x = (08 - 15)
y = Y 25; x = (16 - 23)
y = $Z_1$ 27; x = (16 - 23)
y = Z2 28; x = (16 - 20)
y = $Z_3$ 29; x = (16 - 17)

7. Verwendung des aus den Stufen 1 - 3 des Anspruches 1 erhaltenen, durch vorhergehende Vermehrung der im Blut oder Harn enthaltenen Mikroorganismen auf Blutagarboden bei 35 - 40 °C Bruttemperatur und durch deren Abheben vom Blutagarboden erzeugten Autovaxins zur Herstellung eines Heilmittels.

**Claims**

1. A method for crystal-morphological analysis of blood and urine for early diagnosis and for the preparation of medicaments with the production of a distillate and a calcined material from human blood or excretions with mixing to form a dispersion, with the formation of crystals and comparison with typical crystal patterns of crystal textures and crystal shapes, characterised by the following method steps:
 1) mixing of the human blood or urine or the animal blood or urine from warm-blooded animals with at least three times the quantity of distilled water in a volume ratio of 1 : 2.5 to 3.5, preferably of 1 : 2.7 to 3.2, and distillation without pressure at boiling temperature until a distillate and a practically dry blood cake or urine sediment is formed,
 2) calcination of the dry blood cake or urine sediment rising in linear manner to 600 °C with up to a maximum of 1% dispersion in a heating time of 60 to 70 min, preferably of 65 min, at a constant heating capacity, slow cooling of the calcined material to about 150 °C in about 2 to 4 h, pulverising

18

of the cooled calcined material,

3) mixing of the distillate from stage 1 and the calcined material from stage 2, interpolation of a rest period of about 8 to 16 h to form a homogenous phase, filtration of the liquid mixture,

4) application of the filtered solution at 19.9 to 20.1°C, preferably at 20.0°C, at a relative humidity of about 40 to 60%, to glass microscope slides in the form of 2 drops having a diameter of 6 to 10 mm, preferably of 8 mm, evaporation of the water at 19.9 to 20.1°C, with the formation of practically dry crystal textures with crystal shapes and the association thereof with defined human or animal organs or physiological functional cycles or blastodermic layer sectors, in such a manner that a preferred partial surface in the radial direction in the outer, concentric annular space is associated with the entodermal region (A) and a preferred partial surface in the radial direction in the central, concentric annular space is associated with the mesodermal region (B) and the central annular space being associated with the ectodermal region (C),

5) microscope determination of the crystal textures and crystal shapes in the dark field with

| enlargements | to illustrate |
|---|---|
| 10 to 15 times | the sectors in the total pattern, |
| 20 to 50 times | the individual sectors (A) or (B) or (C) according to stage 4, |
| 60 to 160 times | the compact, plane and/or spatial dendrites as textures, |
| 170 to 800 times | the dendrite patterns with morphological, geometric, spatial basic shapes to identify the crystal-forming basic shapes |

6) microscopic and photo-optical comparison of the crystal textures and crystal shapes defined in stage 5 with those of a significant, pathological condition from comparison series as standard types or with the coordinates of mother and daughter forms for diagnosing the health or illness condition of a human or of vertebrates and mammals.

2.  A method according to Claim 1, characterised in that in stage 1 a quantity of the human or animal blood or of the resin of 3 to 15 ml, preferably of 7 to 10 ml, is used.

3.  A method according to Claims 1 and 2, characterised in that the calcination in stage 2 takes place in three stages at temperature of 200°, 400° and 600°C in such a manner that heating is first carried out in linear manner to 200°C and this temperature is kept constant for 5 to 10 min, and this process is repeated at 400° and at 600°, and then the cooling according to stage 2 takes place.

4.  A method according to Claims 1 to 3, characterised in that the crystal textures and crystal shapes have the following MOTHER FORMS as geometric, morphological figures:

```
Abscissa      Ordinate
x = 0;        y = A 01:  empty circle with maximum diameter
                         of 0.2 mm on the slide,
x = 0;        y = B 02:  solid circle of 0 to a maximum of
                         2 mm diameter on the slide,
x = 0;        y = C 03:  empty circle with small, solid
                         circular area in the interior
                         having any diameter,
x = 0;        y = D 04:  empty circle with diameter greater
                         than 0.2 mm on the slide,
x = 0;        y = E 05:  droplet-like shape with rounding
                         on both long sides,
x = 0;        y = F 06:  elongated oval shape with rounding
                         on both long sides,
```

```
x = 0;          y = G 07: compressed, empty circle with
                           parallel long sides and round
                           corners,
x = 0;          y = H 08: two compressed, empty circles,
x = 0;          y = I 09: two small, slightly compressed,
                           solid circles,
x = 0;          y = K 11: inner circle in a diffuse or
                           geometrically arranged surface,
x = 0;          y = L 12: sinusoidal line,
x = 0;          y = M 13: circular boundary line of a
                           segment,
x = 0;          y = N 14: simple stroke form,
x = 0;          y = O 15: strokes with angled sections,
x = 0;          y = P 16: groups, parallel linear shapes,
x = 0;          y = Q 17: angled and angular shapes,
x = 0;          y = R 18: triangle and triangular shapes,
x = 0;          y = S 19: crossing and intersecting shapes,
x = 0;          y = T 20: quadrilaterals and quadrilateral
                           shapes,
x = 0;          y = U 21: broken rectangular shapes,
x = 0;          y = V 22: fairly large lines with attached
                           and non-bound small, angled line,
x = 0;          y = W 23: fairly large rectangular shapes
                           with smaller, angular shape
                           attached on the outsides,
x = 0;          y = X 24: transverse band shape.
```

5. A method according to Claims 1 to 4, characterised in that the crystal textures and crystal shapes of the daughter forms derived from the mother forms have the following geometric and morphological figures:

| mother forms (1): | abscissa x = 0; | ordinate y = n |
| daughter forms (2): | abscissa x = (01 - m); | ordinate y = n |

| Coordinates | Figurative representation |
|---|---|
| (1) y = A 01<br>(2) x = 01 - 17,<br>　　19, 21 - 26 | Circular shapes with non-solid inner surface, strings, accumulations thereof, of ordered and non-ordered grid formation, |
| (1) y = B 02<br>(2) x = 01 - 19,<br>　　21 - 26 | Circular shapes with solid inner surface, agglomerated, regular or irregular grid formations thereof, and also radial, centrifugal, free or cross-linked attached shapes and ring formations thereof, |
| (1) y = C 03<br>(2) x = 01 - 27 | Circular shapes with denser point-like, laminar, agglomerated, solid inner surface or with radial or crossing or centrifugal single, mixed or internal shapes, |
| (1) y = D 04<br>(2) x = 01 - 16 | Oval and geometric shapes with superposed contour, |
| (1) y = E 05<br>(2) x = 01 - 15,<br>　　21 - 27 | Oval elongated shapes with branches, concavities, radial or flat attached shapes, |
| (1) y = F 06<br>(2) x = 01 - 16,<br>　　18 - 21 | Round, elongated, stretched or branched shapes with pointed ends and empty inner surface, |
| (1) y = H 08<br>(2) x = 01 - 17,<br>　　20 - 27 | Rows, fans, ramifications and geometric grid formations of small, empty, depressed circles, |
| (1) y = I 09 | Rows of small, empty, depressed |

22

| (2) x = | 01 - 19, | circles with ramifications, |
| | 21 - 22 | agglomerations, of small, solid circles, |
| (1) y = | K 11 | Irregular, distributed, negative |
| (2) x = | 01 - 23, 25, 27 | circles in geometrically ordered or non-ordered surfaces, free or filled with geometric shapes, such as the positive images thereof, |
| (1) y = | L 12 | Sinusoidal, simple, linked, |
| (2) x = | 01 - 21, 24 - 27 | crossing, agglomerated shapes, forming geometric grids, |
| (1) y = | M 13 | Curved lines with intersections |
| (2) x = | 01 - 23, 26 | comb-shaped and ladder-shaped shapes, forming geometric grids, |
| (1) y = | N 14 | Simple linear shapes with branches |
| (2) x = | 01 - 25 | on one and both sides, alternate or aligned, |
| (1) y = | O 15 | Simple and bent linear shapes with |
| (2) x = | 01 - 25 | branches on one and both sides, alternate or aligned, |
| (1) y = | P 16 | Groups, double or multiple, |
| (2) x = | 01 - 12, 15 - 16, 20 - 22 | mainly parallel linear shapes, intersecting, mainly parallel linear shapes, |
| (1) y = | Q 17 | Angular linear shapes with |
| (2) x = | 01 - 21 | cluster-like branches and/or with arcuate formations in the limbs of the angles, and also intersections thereof, |
| (1) y = | R 18 | Triangular shapes, |
| (2) x = | 01 - 07, 09 - 12 | |
| (1) y = | S 19 | Simple intersection shapes, and |
| (2) x = | 01 - 15, 21 - 23, 25 | also groupings thereof to form geometric shapes |

```
(1) y = T 20              Quadrilateral and polygonal
(2) x = 01 - 23, 25,      shapes and agglomerates
        27                thereof,
(1) y = U 21              Quadrilateral and polygonal
(2) x = 01 - 04,          shapes with irregular, partially
        09 - 10,          deformed contours,
        16 - 20
(1) y = V 22              Extended linear shapes with free
(2) x = 01 - 17           or crossing arrangement to
                          shorter, extended shapes or to
                          point-like shapes,
(1) y = W 23              Quadrilateral shapes with
(2) x = 01 - 09,          geometric attached shapes on the
        11 - 22           outsides and geometric inclusions
                          in the inner surfaces,
(1) y = X 24              Band shapes consisting of elements
(2) x = 01 - 20           lying transversely or at an angle
                          to the direction of orientation of
                          the bands and the connections
                          thereof or of point-like strings
                          or of periodic, free or geometric
                          shapes,
```

and also the forms according to the coordinates:

| | |
|---|---|
| $y = Y$ 25 | $x = 01 - 23$ |
| $y = Z_0$ 26 | $x = 01 - 10$ |
| $y = Z_1$ 27 | $x = 01 - 23$ |
| $y = Z_2$ 28 | $x = 01 - 20$ |
| $y = Z_3$ 29 | $x = 01 - 13, 16, 17$ |
| $y = Z_4$ 30 | $x = 01 - 15.$ |

6. A method according to Claims 1 to 5, characterised in that the crystal textures and crystal shapes have geometric and morphological figures as coordinates which are complex and/or derived from the mother forms and from the daughter forms, the expression before a bracket representing the superior, figure-forming form and the expression in a bracket representing a subordinate partial form, preferably with the coordinates:

$y = Z_1$ 27; $x = 15$:
   ($y = C$ 03; $x = 09$), ($y = N$ 14; $x = 18$), ($y = V$ 22; $x = 06$)
$y = Z_1$ 27; $x = 16$:
   n($y = D$ 04; $x = 0$), ($y = Q$ 17; $x = 06$, $y = A$ 01; $x = 24$)
$y = Z_1$ 27; $x = 09$:
   ($y = K$ 11; $x = 03$), ($y = T$ 20; $x = 0$, $y = W$ 23; $x = 0$, $y = W$ 23; $x = 17$, $y = C$ 03; $x = 34$, $y = C$ 03; $x = 0$)

EP 0 307 566 B1

$y = Y\ 25;\ x = (01 - 07)$
$y = Z_0\ 26;\ x = (01 - 07)$
$y = Z_1\ 27;\ x = (01 - 07)$
$y = Z_2\ 28;\ x = (01 - 07)$
$y = Z_3\ 29;\ x = (01 - 07)$
$y = Z_4\ 30;\ x = (01 - 07)$
$y = Y\ 25;\ x = (08 - 15)$
$y = Z_0\ 26;\ x = (08 - 10)$
$y = Z_1\ 27;\ x = (08 - 15)$
$y = Z_2\ 28;\ x = (08 - 15)$
$y = Z_3\ 29;\ x = (01 - 07)$
$y = Z_4\ 30;\ x = (08 - 15)$
$y = Y\ 25;\ x = (16 - 23)$
$y = Z_1\ 27;\ x = (16 - 23)$
$y = Z_2\ 28;\ x = (16 - 20)$
$y = Z_3\ 29;\ x = (16 - 17)$.

**7.** The use of the autovaccine produced from stages 1 - 3 of Claim 1, by increasing the microorganisms contained in the blood or urine on a blood agar base at 35 - 40°C incubation temperature and by the removal thereof from the blood agar base, for the preparation of a medicament.

**Revendications**

**1.** Procédé d'analyse cristallo-morphologique de sang et d'urine en vue de diagnostics précoces, et de préparation d'agents médicamenteux par obtention d'un distillat et d'un calcinat de sang humain ou de fractions de sang humain par mélange en dispersion, avec formation de cristallisats et comparaison analogique avec images cristallines typiques de textures cristallines et de formes cristallines, caractérisé par les étapes de procédé suivantes:

1) mélange du sang humain ou de l'urine humaine ou du sang animal ou de l'urine animale d'animaux à sang chaud, avec de l'eau distillée au moins 3 fois, dans un rapport volumique de 1:2,5 à 3,5, et de préférence de 1;2,2 à 3,2, et distillation sous vide à température d'ébullition jusqu'à formation d'un distillat et d'un gâteau ou sédiment de sang pratiquement sec;

2) calcination du gâteau ou sédiment de sang, par augmentation linéaire de la température jusqu'à 600°C, sous recirculation d'au plus 1 %, avec une durée de montée en température de 60 à 70 minutes, et de préférence de 65 minutes, à puissance de chauffe constante, refroidissement lent du calcinat jusqu'à environ 150°C en 2 à 4 heures environ, et réduction en poudre du calcinat refroidi;

3) mélange du distillat provenant de l'étape 2 et du calcinat provenant de l'étape 3, application d'une période de repos de 8 à 16 heures environ, pour formation d'un phase homogène, et filtration du mélange liquide;

4) transfert de la solution filtrée à une température située entre 19,9°C et 20,1°C, et de préférence à 20,0°C, dans une humidité relative d'air de 40 à 60 % environ, sur des porte-objets de microscopie en verre, sous forme de 2 gouttes d'un diamètre de 6 à 10 mm, et de préférence de 8 mm; évaporation de l'eau à une température située entre 19,9°C et 20,1°C, avec formation de structures cristallines pratiquement sèches avec des formes cristallines et un agencement de celles-ci en organes humains ou animaux définis ou en cycles fonctionnels physiologiques ou en secteurs blastodermiques, de telle manière que le domaine endodermal (A) soit associé à une surface préférentielle partielle située sur l'espace annulaire extérieur concentrique, le domaine mésodermal (B) à une surface préférentielle partielle située dans l'espace annulaire médian concentrique, et le domaine ectodermal (C) à l'espace annulaire central,

5) détermination au microscope des textures cristallines et des formes cristallines dans le champ sombre, avec:

| grossissement de | pour définition des |
|---|---|
| 10 à 15 fois<br>20 à 50 fois<br>60 à 160 fois<br>170 à 800 fois | secteurs globaux<br>des secteurs individuels (A), (B), (C) de l'étape 4<br>des textures dendritiques compactes, superficielles et/ou spatiales,<br>des images dendritiques et de leurs formes fondamentales morphologiques et<br>spatiales, en vue de l'identification des formes fondamentales formant les cristaux; |

6) comparaison au microscope ou photo-optique des textures cristallines et formes cristallines définies à l'étape 5 avec celles d'un état pathologique certain provenant des séries comparatives fournissant des types normalisés, ou avec les coordonnées des formes mères et filles, en vue du diagnostic de l'état de santé ou de maladie d'êtres humains ou d'animaux mammifères ou ruminants.

2. Procédé selon la revendication 1 ,**caractérisé en ce qu'**en étape 1 on utilisé une quantité de 3 à 5 ml, et de préférence de 7 à 10 ml, du sang humain ou du sang animal ou de l'urine.

3. Procédé selon les revendications 1 et 2, **caractérisé en ce que** la calcination de l'étape 2 s'effectue en trois phases, à des températures de 200°, 400°, 600°C, de manière à monter en température d'abord linéairement jusqu'à 200°C, que cette température soit maintenue constante pendant 5 à 10 minutes, et que cette procédure soit ensuite reprise à 400°C et à 600°C, et qu'ensuite le refroidissement soit effectué comme en étape 2.

4. Procédé selon les revendications 1 à 3, **caractérisé en ce que** les textures cristallines et les formes cristallines présentent comme FORMES MERES les figures géométriques et morphologiques suivantes:

| Abscisse | Ordonnée | |
|---|---|---|
| x = 0; | y = A 01: | Cercle vide d'un diamètre maximal de 0,2 mm sur le porte-objet. |
| x = 0; | y = B 02: | Cercle plein d'un diamètre maximal de 0 à 2 mm sur le porte-objet. |
| x = 0; | y = C 03: | Cercle vide avec au centre une petite surface circulaire pleine de diamètre quelconque. |
| x = 0; | y = D 04: | Cercle vide avec un diamètre supérieur à 0,2mm sur le porte-objet. |
| x = 0; | y = E 05: | Forme ressemblant à celle d'une goutte, arrondie sur ses deux côtés longitudinaux. |
| x = 0; | y = F 06: | Forme allongée en ovale, arrondie sur ses deux côtés longitudinaux. |
| x = 0; | y = G 07: | Cercle vide aplati avec des côtés longitudinaux parallèles et des coins arrondis. |
| x = 0; | y = H 08: | Deux cercles vides aplatis. |
| x = 0; | y = I 09: | Deux petits cercles pleins légèrement aplatis. |
| x = 0; | y = K 11: | Cercle intérieur dans une surface diffuse ou géométriquement disposée. |
| x = 0; | y = L 12: | Ligne de forme sinusoïdale. |
| x = 0; | y = M 13: | Frontière d'un segment en forme de ligne circulaire. |
| x = 0; | y = N 14: | Simple forme de trait. |
| x = 0; | y = O 15: | Trait formant des arêtes. |
| x = 0; | y = P 16: | Formes linéaires parallèles et découpées. |
| x = 0; | y = Q 17: | Formes anguleuses. |
| x = 0; | y = R 18: | Triangles et formes triangulaires. |
| x = 0; | y = S 19: | Croix et formes se croisant. |
| x = 0; | y = T 20: | Quadrangles et formes quadrangulaires. |
| x = 0; | y = U 21: | Formes quadrangulaires rompues. |
| x = 0; | y = V 22: | Grandes lignes avec ligne plus petite, raccordée ou non, et formant des arêtes. |
| x = 0; | y = W 23: | Grandes formes rectangulaires avec dépôt anguleux plus petit sur les bords extérieurs. |
| x = 0; | y = X 24: | Bandes se croisant. |

5. Procédé selon les revendications 1 à 4, **caractérisé en ce que** les textures cristallines et les formes cristallines des formes filles déduites des formes mères présentent les figures morphologiques et géométriques suivantes:

| formes mères (1): | abscisse x = 0 | ordonnée y = n |
|---|---|---|
| formes filles (2): | abscisse x = (01 - m) | ordonnée y = n |

| Coordonnées | Détermination de la figure |
|---|---|
| (1) y = A 01 | Formes circulaires dont la surface intérieure n'est |
| (2) x = 01 - 17, 19, | pas remplie, ainsi que leurs séries, accumulations |
| 21 - 26 | et réseaux, ordonnés ou non. |
| | |
| (1) y = B 02 | Formes circulaires dont la surface intérieure est |
| (2) x = 01 - 19, | remplie, ainsi que leurs réseaux empilés de |
| 21 - 26 | manière régulière ou non et que leurs accumulations |
| | radiales, centrifuges, annulaires, libres ou réticulées. |
| | |
| (1) y = C 03 | Surfaces intérieures pleines en forme de point, |
| (2) x = 01 - 27 | étendues, répétées, ou avec des formes radiales, se |
| | croisant ou centrifuges encloses, ou se mélangeant, |
| | ou indépendantes. |
| | |
| (1) y = D 04 | Formes ovales et géométriques aux contours |
| (2) x = 01 - 16 | superposés. |
| | |
| (1) y = E 05 | Formes ovales allongées, avec bifurcations, |
| (2) x = 01 - 15, | dentelures, accumulations radiales ou superficielles. |
| 21 - 27 | |
| | |
| (1) y = F 06 | Formes arrondies, allongées, étendues ou à |
| (2) x = 01 - 16, | bifurcations, à extrémités pointues et surface |
| 18 - 21, | intérieure vide. |

23 - 24

(1) y = H 08        Séries, ensembles, bifurcations et réseaux

(2) x = 01 - 17,       géométriques de petits cercles vides aplatis.

        20 - 27

(1) y = I 09        Séries de petits cercles vides aplatis avec

(2) x = 01 - 19,       bifurcations, accumulations de petits cercles

        21 - 22           pleins.

(1) y = K 11        Cercles en négatif irréguliers et divisés, en

(2) x = 01 - 23,       surfaces géométriques régulières ou non, libres

        25, 27           ou remplis de formes géométriques, ainsi que leurs

formes en positif.

(1) y = L 12        Formes répétées sinusoïdales, simples ou réticulées,

(2) x = 01 - 21,       croisées et formant une grille géométrique.

(1) y = M 13        Lignes courbes se croisant, formes en peigne ou en

(2) x = 01 - 23, 26     échelle formant un réseau géométrique.

(1) y = N 14        Formes linéaires simples avec bifurcations

(2) x = 01 - 25        unilatérales ou bilatérales alternées ou opposées.

(1) y = O 15        Formes linéaires simples et cintrées, avec

(2) x = 01 - 25        bifurcations unilatérales ou bilatérales alternées ou

opposées.

(1) y = P 16        Formes linéaires en ciseaux doubles ou simples, le

(2) x = 01 - 12,       plus souvent parallèles, formes linéaires croisées, le

        15 - 16,       plus souvent parallèles.

        20 - 22

(1) y = Q 17        Formes linéaires anguleuses avec bifurcations en

(2) x = 01 - 21        forme de touffes et/ou avec forme cintrée des côtés

des angles, ainsi que les croisements de ces formes.

(1) y = R 18            Formes triangulaires

(2) x = 01 - 07,

      09 - 12

(1) y = S 19            Formes en simple croix, ainsi que leurs

(2) x = 01 - 15,          accumulations en formes géométriques.

      21 - 23

(1) y = T 20            Formes quadrangulaires et polygones à plus de 4

(2) x = 01 - 15,          côtés, ainsi que leurs agglomérats.?

      21 - 23, 25

(1) y = U 21            Formes quadrangulaires ou polygones à plus de 4

(2) x = 01 - 04,          côtés dont les contours sont partiellement et

      09 - 10,            irrégulièrement déformés

      16 - 20

(1) y = V 22            Formes linéaires allongées avec agencement libre ou

(2) x = 01 - 17           croisé de formes allongées plus courtes ou de

                             formes ponctuelles.   .

(1) y = W 23           Formes quadrangulaires avec dépôts géométriques

(2) x = 01 - 09,          sur les côtés extérieurs et inclusions géométriques

      11 - 22            à l'intérieur.

(1) y = X 24           Rubans formés d'éléments situés transversalement

(2) x = 01 - 20          ou suivant un angle par rapport à l'axe du ruban,

                             ainsi que par leurs associations ou empilements

                             ponctuels, ou formés de formes libres ou

                             géométriques périodiques,

ainsi que les formes correspondant aux coordonnées:

| $y = Y\ 25$ | $x = 01 - 23$ |
|---|---|
| $y = Z_0\ 26$ | $x = 01 - 10$ |
| $y = Z_1\ 27$ | $x = 01 - 23$ |
| $y = Z_2\ 28$ | $x = 01 - 20$ |
| $y = Z_3\ 29$ | $x = 01 - 13, 16, 17$ |
| $y = Z_4\ 30$ | $x = 01 - 15.$ |

**6.** Procédé selon les revendications 1 à 5, **caractérisé en ce que** les textures de cristallisation et les formes de cristallisation présentent comme coordonnées des figures géométriques et morphologiques complexes et/ou déduites des formes mères et des formes filles, l'expression précédent une parenthèse représentant la forme de base associée à la figure et l'expression entre parenthèses présentant une forme partielle subordonnée, et ce de préférence avec les coordonnées:

$y = Z_1$ 27; $x$ = 15:

 ($y$ = C 03; $x$ = 09), $y$ = N 14; $x$ = 18), $y$ = V 22; $x$ = 06)

$y = Z_1$ 27; $x$ = 16:

 n($y$ = D 04; $x$ = O), ($y$ = Q 17; $x$ = 06), $y$ = A 01; $x$ = 24)

$y = Z_1$ 27; $x$ = 09:

 ($y$ = K 11; $x$ = 03), ($y$ = T 20; $x$ = 0, $y$ = X 23; $x$ = 0, $y$ = W 23;

 $x$ = 17, $y$ = C 03; $x$ = 34, $y$ = C 03; $x$ = 0) $y$ = Y 25 $x$ = (01 - 07)

$y = Z_0$ 26 $x$ = (01 - 07)

$y = Z_1$ 27 $x$ = (01 - 07)

$y = Z_2$ 28 $x$ = (01 - 07)

$y = Z_3$ 29 $x$ = (01 - 07)

$y = Z_4$ 30 $x$ = (01 - 07)

$y$ = Y 25 $x$ = (08 - 15)

$y = Z_0$ 26 $x$ = (08 - 10)

$y = Z_1$ 27 $x$ = (08 - 15)

$y = Z_2$ 28 $x$ = (08 - 15)

$y = Z_3$ 29 $x$ = (01 - 07)

$y = Z_4$ 30 $x$ = (08 - 15)

$y$ = Y 25 $x$ = (16 - 23)

$y = Z_1$ 27 $x$ = (16 - 23)

$y = Z_2$ 28 $x$ = (16 - 20)

$y = Z_3$ 29 $x$ = (16 - 17)

**7.** Recours, en vue de la préparation d'un médicament, à l'autovaccin obtenu à partir de la multiplication, sur support d'agar additionné de sang, à une température de sang de 35 -40°C, des micro-organismes contenus dans le sang ou l'urine et provenant des étapes 1 - 3 de la revendication 1, en les extrayant à cet effet de l'agar-agar additionné de sang.

Figur 1

Figur 2

EP 0 307 566 B1

Figur 3

Figur 4

Figur 5

EP 0 307 566 B1

Figur 6

Figur 7

Figur 8

Figur 9

EP 0 307 566 B1

Figur 10

Figur 11

EP 0 307 566 B1

Figur 12

Y

Q 17
R 18
S 19
T 20
μ 21
V 22
ω 23
X 24

24    25    26    27    x

Figur 13

Figur 14

Figur 13.

EP 0 307 566 B1

Figur 16

Figur 17

Figur 18

Figur 19

Entoderm
Kopfhöhlen
Zähne
Rachen/Mandeln
Lymphe
Lungen
Magen/Duoden
Dünndarm
Dickdarm
Mastdarm
Leber
Blase
Uterus/Prostata

Mesoderm

Pankreas
Bindegewebe
Muskulatur
Knochen
Gelenke
Nieren
Herz

Ektoderm

Endokrinium
Haut
Zentrales/
peripheres
Nervensystem

Abbildung 1

Abbildung 2

Abbildung 3

Abbildung 4

Abbildung 5

Abbildung 6

Abbildung 7

Abbildung 8

Abbildung 9

Abbildung 10

Abbildung 11

Abbildung 12

Abbildung 13

Abbildung 14

Abbildung 15

Abbildung 16

Abbildung 17

Abbildung 18

Abbildung 19

Abbildung 20

Abbildung 21

Abbildung 22

Abbildung 23

Abbildung 24

Abbildung 25

55

Abbildung 26

Abbildung 27

Abbildung 28

Abbildung 29

Abbildung 30